# EUROPEAN PATENT APPLICATION

(11) **EP 1 589 001 A1**
(43) Date of publication of application: **26.10.2005**
(21) Application number: 04290981.2
(22) Date of filing: 13.04.2004
(51) Int. Cl.: C07C 317/44, C07C 323/60, C07D 333/22, C07D 333/18, C07D 307/38, C07D 249/12

(54) **Tricyclic aromatic and bis-phenyl sulfinyl derivatives**

(71) Applicant: CEPHALON, INC., West Chester, PA 19380-4245 (US)
(72) Inventor: Bacon, Edward E., Audubon, PA 19403 (US); Chatterjee, Sankar, Wynnewood, PA 19096 (US); Dunn, Derek, Coatesville, PA 19320 (US); Gruner, John A., West Chester, PA 19382 (US); Tripathy, Rabindranath, Churchville, PA 18966 (US)
(74) Representative: Bernasconi, Jean Raymond

(57) **Abstract**

The present invention provides compounds of the structure: 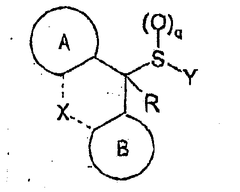 including pharmaceutical compositions thereof and methods of treating diseases such as for example excessive sleepiness, promotion and/or improvement of wakefulness.

## Description

### FIELD OF THE INVENTION

The present invention is related to chemical compositions, processes for the preparation thereof and uses of the composition. Particularly, the present invention relates to compositions that include substituted thioacetamides, and their use in the treatment of diseases, such as excessive sleepiness, promotion and/or improvement of wakefulness (preferably improvement of wakefulness in patients with excessive sleepiness associated with narcolepsy, sleep apnea (preferably obstructive sleep apnea/hypopnea) and shift work disorder), treatment of Parkinson's disease, Alzheimer's disease, cerebral ischemia, stroke, eating disorders, attention deficit disorder ("ADD"), attention deficit hyperactivity disorder ("ADHD"), depression, schizophrenia, fatigue (preferably fatigue associated with cancer or neurological diseases, such as multiple sclerosis and chronic fatigue syndrome), stimulation of appetite and weight gain and improvement of cognitive dysfunction.

### BACKGROUND OF THE INVENTION

The compounds disclosed herein are related to the biological and chemical analogs of modafinil. Modafinil, C₁₅H₁₅NO₂S, also known as 2-(benzhydrylsulfinyl) acetamide, or 2-[(diphenylmethyl) sulfinyl] acetamide, a synthetic acetamide derivative with wake-promoting activity, has been described in French Patent No. 78 05 510 and in U.S. Patent No. 4,177,290 ("the '290 patent"). It has been approved by the United States Food and Drug Administration for use in the treatment of excessive daytime sleepiness associated with narcolepsy. Methods for preparing modafinil and several derivatives are described in the '290 patent. The levorotatory isomer of modafinil, along with additional modafinil derivatives are described in U.S. Patent No. 4,927,855, and are reported to be useful for treatment of hypersomnia, depression, Alzheimer's disease and to have activity towards the symptoms of dementia and loss of memory, especially in the elderly.

Modafinil has also been described as a useful agent in the treatment of Parkinson's disease (U.S. Patent No. 5,180,745); in the protection of cerebral tissue from ischemia (U.S. Patent No. 5,391,576); in the treatment of urinary and fecal incontinence (U.S. Patent No. 5,401,776); and in the treatment of sleep apneas and disorders of central origin (U.S. Patent No. 5,612,379). In addition, modafinil may be used in the treatment of eating disorders, and to promote weight gain or stimulate appetite in humans or animals (U.S. Patent No. 6,455,588), and in the treatment of attention deficit hyperactivity disorder (U.S. Patent No. 6,346,548), and fatigue, especially fatigue associated with multiple sclerosis (US Patent No. 6,488,164). U.S. Pat. No. 4,066,686 describes various benzhydrylsulphinyl derivatives as being useful in therapy for treating disturbances of the central nervous system.

Several published patent applications describe derivative forms of modafinil and the use of modafinil derivatives in the treatment of various disorders. For example, PCT publication WO 99/25329 describes various substituted phenyl analogs of modafinil as being useful for treating drug-induced sleepiness, especially sleepiness associated with administration of morphine to cancer patients. U.S. Pat No. 5,719,168 and PCT Publication No. 95/01171 describes modafinil derivatives that are useful for modifying feeding behavior. PCT Publication No. 02/10125 describes several modafinil derivatives of modafinil, along with various polymorphic forms of modafinil.

Additional publications describing modafinil derivatives include U.S. Pat. No. 6,492,396, and PCT Publ. No. WO 02/10125.

Terauchi, H, et al. described nicotinamide derivatives useful as ATP-ase inhibitors (Terauchi, H, et al, *J. Med. Chem*., **1997,** *40,* 313-321). In particular, several N-alkyl substituted 2-(Benzhydrylsulfinyl) nicotinamides are described.

U.S. Pat. Nos. 4,980,372 and 4,935,240 describe benzoylaminophenoxybutanoic acid derivatives. In particular, sulfide derivatives of modafinil containing a phenyl and substituted phenyl linker between the sulfide and carbonyl, and a substituted aryl in the terminal amide position, are disclosed.

Other modafinil derivatives have been disclosed wherein the terminal phenyl groups are constrained by a linking group. For example, in U.S. Pat. No. 5,563,169, certain xanthenyl and thiaxanthenyl derivatives having a substituted aryl in the terminal amide position are reported.

Other xanthenyl and thiaxanthenyl derivatives are disclosed in Annis, I; Barany, G. *Pept. Proc. Am. Pept. Symp. 15*^{*th*} (Meeting Date 1997) 343-344, 1999 (preparation of a xanthenyl derivative of Ellman's Reagent, useful as a reagent in peptide synthesis); Han, Y.; Barany, G. *J. Org. Chem.,* **1997,** *62*, 3841-3848 (preparation of S-xanthenyl protected cysteine derivatives, useful as a reagent in peptide synthesis); and El-Sakka, I.A., et al. *Arch. Pharm. (Weinheim),* **1994,** *327*, 133-135 (thiaxanthenol derivatives of thioglycolic acid).

Thus, there is a need for novel classes of compounds that possess the beneficial properties similar to that of modafinil. It has been discovered that a class of compounds, referred to herein as substituted thioacetamides, are useful as agents for treating or preventing various diseases or disorders disclosed herein.

### SUMMARY OF THE INVENTION

The present invention in one aspect is directed to novel compounds which are useful in the treatment of diseases, such as excessive sleepiness, promotion and/or improvement of wakefulness (preferably improvement of wakefulness in patients with excessive sleepiness associated with narcolepsy, sleep apnea (preferably obstructive sleep apnea/hypopnea) and shift work disorder), treatment of Parkinson's disease, Alzheimer's disease, cerebral ischemia, stroke, eating disorders, attention deficit disorder ("ADD"), attention deficit hyperactivity disorder ("ADHD"), depression, schizophrenia, fatigue (preferably fatigue associated with cancer or neurological diseases, such as multiple sclerosis and chronic fatigue syndrome), stimulation of appetite and weight gain and improvement of cognitive dysfunction.

These compounds have the structure: 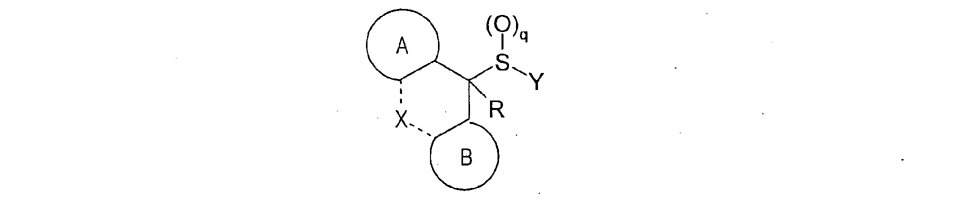 and its stereoisomeric forms, mixtures of stereoisomeric forms, or pharmaceutically acceptable salt forms thereof, wherein the constituent members are defined *infra*.

In another aspect, the present invention is directed to a pharmaceutical composition which comprises a pharmaceutically acceptable carrier and a therapeutically effective amount of a compound of the present invention.

In yet another aspect, the present invention is directed to methods of preventing or treating the diseases or disorders disclosed herein.

### DETAILED DESCRIPTION OF THE INVENTION

In one aspect of the present invention there are provided compounds of formula (A) for the utilities provided herein: 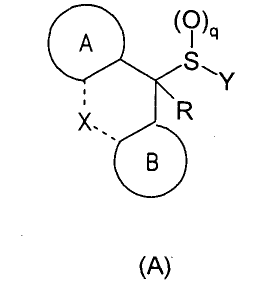 wherein
rings A and B, together with the carbon atoms to which they are attached, are each independently selected from:
a) a 6-membered aromatic carbocyclic ring in which from 1 to 3 carbon atoms may be replaced by hetero atoms selected from oxygen, nitrogen and sulfur; and
b) a 5-membered aromatic carbocyclic ring in which either:
   i) one carbon atom may be replaced with an oxygen, nitrogen, or sulfur atom;
   ii) two carbon atoms may be replaced with a sulfur and a nitrogen atom, an oxygen and a nitrogen atom, or two nitrogen atoms; or
   iii) three carbon atoms may be replaced with three nitrogen atoms, one oxygen and two nitrogen atoms, or one sulfur and two nitrogen atoms;
wherein said rings are optionally substituted with one to three R²⁰ groups;
X is not present, is a bond, O, S(O)_{y}, NR¹⁰, C₂ alkylene, C₂₋₃ alkenylene, C(=O), C(R²¹)₂NR¹⁰, C(R²¹)=N, N=C(R²¹), C(=O)N(R¹⁰), or NR¹⁰C(=O); wherein said alkylene and alkenylene groups are optionally substituted with one to three R²⁰ groups;
R is H, C₁-C₆ alkyl, C₆-C₁₀ aryl, 5-6 membered heteroaryl, C₃-C₇ cycloalkyl, or 3-7 membered heterocycloalkyl; with the proviso that R cannot be H when R¹ is C(=O)NR¹²R¹³;
Y is C₁-C₉ alkylene-R¹, wherein one or two carbon atoms can be replaced by one or two O, NR¹⁰, or S(O)_{y} groups, or a carbon atom can be replaced by a C₆-C₁₀ arylene, 5-10 membered heteroarylene, C₃-C₆ cycloalkylene, or 3-6 membered heterocycloalkylene group; C₂-C₆ alkenylene-R¹; or C₂-C₆ alkynylene-R¹; wherein said alkylene, alkenylene, alkynylene, arylene, heteroarylene, cycloalkylene, and heterocycloalkylene groups are optionally substituted with one to three R²⁰ groups;
R¹ is selected from H, NR¹²R¹³, NR²¹C(=O)R¹⁴, C(=O)R¹⁵, CO₂R¹¹, OC(=O)R¹¹, C(=O)NR¹²R¹³, C(=O)NR²¹OR¹⁴, C(=NR¹¹)NR¹²R¹³, NR²¹S(O)₂R¹¹, S(O)₂NR¹²R¹³, NR²¹S(O)₂NR¹²R¹³, and PO(OR²¹)₂;
R¹⁰ and R^{10A} at each occurrence are independently selected from H, C₁-C₆ alkyl, C₆-C₁₀ aryl, C(=O)R¹⁵, and S(O)_{y}R¹⁴; wherein said alkyl and aryl groups are optionally substituted with one to three R²⁰ groups;
R¹¹ at each occurrence is independently selected from H, and C₁-C₆ alkyl; wherein said alkyl is optionally substituted with one to three R²⁰ groups;
R¹² and R¹³ at each occurrence are each independently selected from H, C₁-C₆ alkyl, and C₆-C₁₀ aryl, or R¹² and R¹³, together with the nitrogen to which they are attached, form a 3-7 membered heterocycloalkyl ring;
wherein said alkyl and aryl groups and heterocycloalkyl ring are optionally substituted with one to three R²⁰ groups;
R¹⁴ at each occurrence is independently selected from C₁-C₆ alkyl, C₆-C₁₀ aryl, and arylalkyl; wherein said alkyl, aryl and arylalkyl groups are optionally substituted with one to three R²⁰ groups;
R¹⁵ at each occurrence is independently selected from C₁-C₆ alkyl, C₆-C₁₀ aryl, arylalkyl, and heteroaryl; wherein said alkyl, aryl, arylalkyl, and heteroaryl groups are optionally substituted with one to three R²⁰ groups;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocycloalkyl ring;
R²⁵ at each occurrence is independently the residue of an amino acid after the hydroxyl group of the carboxyl group is removed;
m is 0 or 1;
n is 0 or 1;
q is 0, 1, or 2;
y is 0, 1, or 2;
with the proviso that when R = H and Y is (C₁-C₆ alkylene)-C(=O)NR¹²R¹³, then the alkylene group must be substituted with a spirocycloalkyl group;
and the stereoisomeric forms, mixtures of stereoisomeric forms or pharmaceutically acceptable salts forms thereof.

In an additional aspect of the present invention there are provided compounds of formula (I): 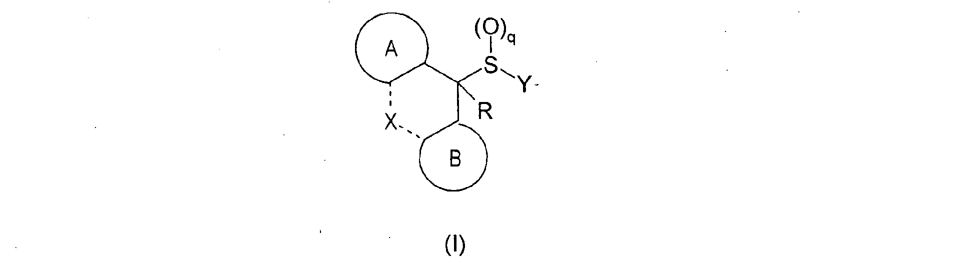 wherein
rings A and B, together with the carbon atoms to which they are attached, are each independently selected from:
a) a 6-membered aromatic carbocyclic ring in which from 1 to 3 carbon atoms may be replaced by hetero atoms selected from oxygen, nitrogen and sulfur; and
b) a 5-membered aromatic carbocyclic ring in which either:
   i) one carbon atom may be replaced with an oxygen, nitrogen, or sulfur atom;
   ii) two carbon atoms may be replaced with a sulfur and a nitrogen atom, an oxygen and a nitrogen atom, or two nitrogen atoms; or
   iii) three carbon atoms may be replaced with three nitrogen atoms, one oxygen and two nitrogen atoms, or one sulfur and two nitrogen atoms;
wherein said rings are optionally substituted with one to three R²⁰ groups;
X is not present, is a bond, O, S(O)_{y}, NR¹⁰, C₂ alkylene, C₂₋₃ alkenylene, C(=O), C(R²¹)₂NR¹⁰, C(R²¹)=N, N=C(R²¹), C(=O)N(R¹⁰), or NR¹⁰C(=O); wherein said alkylene and alkenylene groups are optionally substituted with one to three R²⁰ groups;
R is H, C₁-C₆ alkyl;
Y is selected from:
a) C₁-C₆ alkylene-R¹;
b) C₁-C₆ alkylene-R²;
c) (C₁-C₄ alkylene)ₘ-Z-(C₁-C₄ alkylene)ₙ-R¹;
d) C₁-C₆ alkylene-O(CH₂)ₚOR²¹,
e) C₁-C₆ alkyl substituted with one or two OR¹¹ groups; provided that Y cannot be (CH₂)₁₋₄OR¹¹; and
f) CH₂CR²¹=C(R²¹)₂ except when X is a bond and q is 2;
wherein said alkyl and alkylene groups are optionally substituted with one to three R²⁰ groups;
Z is O, NR^{10A}, S(O)_{y}, CR²¹=CR²¹, C=C(R²¹)₂, C=C, C₆-C₁₀ arylene, 5-10 membered heteroarylene, C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
R¹ is selected from NR¹²R¹³, NR²¹C(=O)R¹⁴, C(=O)R¹⁵; CO₂R¹¹, OC(=O)R¹¹, C(=O)NR¹²R¹³, C(=O)NR²¹OR¹⁴, C(=NR¹¹)NR¹²R¹³, NR²¹S(O)₂R¹¹, S(O)₂NR¹²R¹³, NR²¹S(O)₂NR¹²R¹³, and PO(OR²¹)₂;
R² is a 5-6 membered heteroaryl, wherein said heteroaryl group is optionally substituted with one to three R²⁰ groups;
R¹⁰ and R^{10A} at each occurrence are independently selected from H, C₁-C₆ alkyl, C₆-C₁₀ aryl, C(=O)R¹⁵, and S(O)_{y}R¹⁴; wherein said alkyl and aryl groups are optionally substituted with one to three R²⁰ groups;
R¹¹ at each occurrence is independently selected from H and C₁-C₆ alkyl; wherein said alkyl is optionally substituted with one to three R²⁰ groups;
R¹² and R¹³ at each occurrence are each independently selected from H, C₁-C₆ alkyl, and C₆-C₁₀ aryl, or R¹² and R¹³, together with the nitrogen to which they are attached, form a 3-7 membered heterocycloalkyl ring;
wherein said alkyl and aryl groups and heterocycloalkyl ring are optionally substituted with one to three R²⁰ groups;
R¹⁴ at each occurrence is independently selected from C₁-C₆ alkyl, C₆-C₁₀ aryl, and arylalkyl; wherein said alkyl, aryl and arylalkyl groups are optionally substituted with one to three R²⁰ groups;
R¹⁵ at each occurrence is independently selected from C₁-C₆ alkyl, C₆-C₁₀ aryl, arylalkyl, and heteroaryl; wherein said alkyl, aryl, arylalkyl, and heteroaryl groups are optionally substituted with one to three R²⁰ groups;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocycloalkyl ring;
R²⁵ at each occurrence is independently the residue of an amino acid after the hydroxyl group of the carboxyl group is removed;
m is 0 or 1;
n is 0 or 1;
p is 1, 2, 3, or 4;
q is 0, 1, or 2;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
2) when X is not present, then R¹ cannot be NR¹²R¹³;
and the stereoisomeric forms, mixtures of stereoisomeric forms or pharmaceutically acceptable salts forms thereof.

In yet another embodiment of the present invention there are provided compounds of formula (II): 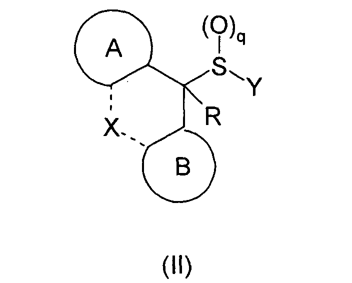 wherein
rings A and B, together with the carbon atoms to which they are attached, are each independently selected from phenylene, pyridylene, thienylene, or a 5-membered aromatic ring in which one or two carbon atoms may be replaced with a nitrogen atom;
wherein said rings are optionally substituted with one to three R²⁰ groups;
X is not present, is a bond, O, S(O)_{y}, NR¹⁰, C₂ alkylene, or C₂ alkenylene, wherein said alkylene and alkenylene groups are optionally substituted with one to three R²⁰ groups;
R is H or C₁-C₆ alkyl;
Y is selected from:
a) C₁-C₆ alkylene-R¹;
b) C₁-C₆ alkylene-R²;
c) (C₁-C₄ alkylene)ₘ-Z¹-(C₁-C₄ alkylene)ₙ-R¹, or
   C₁-C₄ alkylene-Z²-C₁-C₄ alkylene-R¹;
d) C₁-C₆ alkylene-O(CH₂)ₚOR²¹,
e) C₁-C₆ alkyl substituted with one or two OR¹¹ groups; provided that Y cannot be (CH₂)₁₋₄OR¹¹;
f) CH₂CR²¹=C(R²¹)₂ CH₂CR²¹=C(R²¹)₂ except when X is a bond and q is 2;
wherein said alkyl and alkylene groups are optionally substituted with one to three R²⁰ groups;
Z¹ is CR²¹=CR²¹, C=C(R²¹)₂, C=C, phenylene, 5-6 membered heteroarylene, C₃-C₆ cycloalkylene, or 5-6 membered heterocycloalkylene; wherein said phenylene, heteroarylene, cycloalkylene, and heterocycloalkylene groups are optionally substituted with one to three R²⁰ groups;
Z² is O, NR^{10A}, S(O)_{y};
R¹ is selected from NR¹²R¹³, NR²¹C(=O)R¹⁴, C(=O)R¹⁵, CO₂R¹¹, OC(=O)R¹¹, C(=O)NR¹²R¹³, C(=O)NR²¹OR¹⁴, C(=NR¹¹)NR¹²R¹³, NR²¹S(O)₂R¹¹, S(O)₂NR¹²R¹³, NR²¹S(O)₂NR¹²R¹³, and PO(OR²¹)₂;
R² is a 5-6 membered heteroaryl, wherein said heteroaryl group is optionally substituted with one to three R²⁰ groups;
R¹⁰ and R^{10A} at each occurrence are independently selected from H, C₁-C₆ alkyl, C₆-C₁₀ aryl, C(=O)R¹⁵, and S(O)_{y}R¹⁴; wherein said alkyl and aryl groups are optionally substituted with one to three R²⁰ groups;
R¹¹ at each occurrence is independently selected from H, and C₁-C₆ alkyl; wherein said alkyl is optionally substituted with one to three R²⁰ groups;
R¹² and R¹³ at each occurrence are each independently selected from H, C₁-C₆ alkyl, and C₆-C₁₀ aryl, or R¹² and R¹³, together with the nitrogen to which they are attached, form a 3-7 membered heterocycloalkyl ring;
wherein said alkyl and aryl groups and heterocycloalkyl ring are optionally substituted with one to three R²⁰ groups;
R¹⁴ at each occurrence is independently selected from C₁-C₆ alkyl, C₆-C₁₀ aryl, and arylalkyl; wherein said alkyl, aryl and arylalkyl groups are optionally substituted with one to three R²⁰ groups;
R¹⁵ at each occurrence is independently selected from C₁-C₆ alkyl, C₆-C₁₀ aryl, arylalkyl, and heteroaryl; wherein said alkyl, aryl, arylalkyl, and heteroaryl groups are optionally substituted with one to three R²⁰ groups;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocycloalkyl ring;
R²⁵ at each occurrence is independently the residue of an amino acid after the hydroxyl group of the carboxyl group is removed;
m is 0 or 1;
n is 0 or 1;
p is 1, 2, 3, or 4;
q is 0, 1, or 2;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
2) when X is not present, then R¹ cannot be NR¹²R¹³;
and the stereoisomeric forms, mixtures of stereoisomeric forms or pharmaceutically acceptable salts forms thereof.

In yet another embodiment of the present invention there are provided compounds of formula (III): 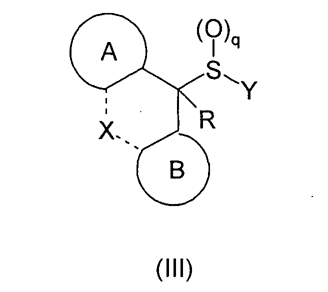 wherein
rings A and B, together with the carbon atoms to which they are attached, are each independently selected from phenylene, pyridylene, furylene, thienylene, or a 5-membered aromatic ring in which 1-3 carbon atoms may be replaced with a nitrogen atom;
wherein said rings are optionally substituted with one to three R²⁰ groups;
X is not present, is a bond, O, S(O)_{y}, NR¹⁰, wherein said alkylene and alkenylene groups are optionally substituted with one to three R²⁰ groups;
R is H or C₁-C₄ alkyl;
Y is selected from:
a) C₁-C₆ alkylene-R¹;
b) C₁-C₆ alkylene-R²;
c) (C₁-C₄ alkylene)ₘ-Z-(C₁-C₄ alkylene)ₙ-R¹;
d) C₁-C₆ alkylene-O(CH₂)ₚOR²¹,
e) CH₂C(OH)(CH₃)₂, CH₂C(CH₃)₂OH, CH₂C(OH)₂CF₃, CH₂C(OH)(C≡CH)₂, or CH₂CH(OH)CH₃,
f) CH₂CR²¹=C(R²¹)₂, or CH₂CR²¹=C(R²¹)₂ except when X is a bond and q is 2;
wherein said alkyl and alkylene groups are optionally substituted with one to three R²⁰ groups;
Z¹ is CR²¹=CR²¹, C=C(R²¹)₂, C=C, or phenylene; wherein said phenylene group is optionally substituted with one to three R²⁰ groups;
Z² is O, NR^{10A}, S(O)_{y};
R¹ is selected from NR¹²R¹³, NR²¹C(=O)R¹⁴, C(=O)R¹⁵, CO₂R¹¹, C(=O)NR¹²R¹³, C(=O)NR²¹OR¹⁴, NR²¹S(O)₂R¹¹, S(O)₂NR¹²R¹³, and PO(OR²¹)₂;
R² is pyridyl, furyl, thienyl, or a 5-membered heteroaryl group containing 1-3 nitrogen atoms;
wherein said heteroaryl group is optionally substituted with one to three R²⁰ groups;
R¹⁰ and R^{10A} at each occurrence are independently selected from H, C₁-C₆ alkyl, C₆-C₁₀ aryl, C(=O)R¹⁵, and S(O)_{y}R¹⁴; wherein said alkyl and aryl groups are optionally substituted with one to three R²⁰ groups;
R¹¹ at each occurrence is independently selected from H, and C₁-C₆ alkyl; wherein said alkyl is optionally substituted with one to three R²⁰ groups;
R¹² and R¹³ at each occurrence are each independently selected from H and C₁-C₆ alkyl, or R¹² and R¹³, together with the nitrogen to which they are attached, form a 5-6 membered heterocycloalkyl;
wherein said alkyl and heterocycloalkyl groups are optionally substituted with one to three R²⁰ groups;
R¹⁴ at each occurrence is independently selected from C₁-C₆ alkyl, C₆-C₁₀ aryl, and arylalkyl; wherein said alkyl, aryl and arylalkyl groups are optionally substituted with one to three R²⁰ groups;
R¹⁵ at each occurrence is independently selected from C₁-C₆ alkyl, C₆-C₁₀ aryl, arylalkyl, and heteroaryl; wherein said alkyl, aryl, arylalkyl, and heteroaryl groups are optionally substituted with one to three R²⁰ groups;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and phenyl;
R²³ and R²⁴ at each occurrence are each independently selected from H and C₁-C₆ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
m is 0 or 1;
n is 0 or 1;
p is 1, 2, 3, or 4;
q is 0, 1, or 2;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
2) when X is not present, then R¹ cannot be NR¹²R¹³;
and the stereoisomeric forms, mixtures of stereoisomeric forms or pharmaceutically acceptable salts forms thereof.

In a further embodiment of the present invention there are provided compounds of formula (IV): 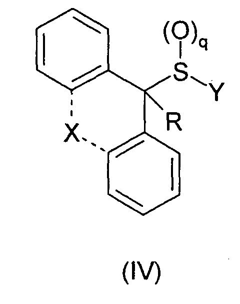 wherein
the phenylene rings are each independently optionally substituted with one to three R²⁰ groups;
X is not present or is a bond;
R is H or C₁-C₄ alkyl;
Y is selected from:
a) C₁-C₆ alkylene-R¹;
b) C₁-C₆ alkylene-R²;
c) C₁-C₄ alkylene-O(CH₂)ₚOR²¹,
d) CH₂C(OH)(CH₃)₂, CH₂C(CH₃)₂OH, CH₂C(OH)₂CF₃, CH₂C(OH)(C≡CH)₂, or CH₂CH(OH)CH₃,
e) CH₂CR²¹=C(R²¹)₂ CH₂CR²¹=C(R²¹)₂ except when X is a bond and q is 2;
wherein said alkylene groups optionally substituted with an R²⁰ group;
R¹ is selected from pyrrolidinyl, piperidinyl, morpholinyl, NR²¹C(=O)R¹⁴, C(=O)R¹⁵, CO₂R¹¹, C(=O)NR¹²R¹³, C(=O)NR²¹OR¹⁴, NR²¹S(O)₂R¹¹, S(O)₂NR^{12A}R^{13A}, and PO(OR²¹)₂;
R² is furyl, thienyl, a 5-membered heteroaryl group containing 1-2 nitrogen atoms, or triazolyl;
wherein said R² groups are optionally substituted with an R²⁰ group;
R¹¹ at each occurrence is independently C₁-C₆ alkyl;
R¹² and R¹³ at each occurrence are each independently selected from H and C₁-C₆ alkyl, or R¹² and R¹³, together with the nitrogen to which they are attached, form a 5-6 membered heterocycloalkyl;
wherein said alkyl and heterocycloalkyl groups are optionally substituted with an R²⁰ group;
R^{12A} and R^{13A} at each occurrence are each independently selected from H and C₁-C₆ alkyl;
R¹⁴ at each occurrence is independently C₁-C₆ alkyl;
R¹⁵ at each occurrence is independently selected from C₁-C₆ alkyl, and 5-membered heteroaryl;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and phenyl;
R²³ and R²⁴ at each occurrence are each independently selected from H and C₁-C₆ alkyl, or
R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
p is 1, 2, 3, or 4;
q is 1 or 2;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
2) when X is not present, then R¹ cannot be pyrrolidinyl, piperidinyl, or morpholinyl;
and the stereoisomeric forms, mixtures of stereoisomeric forms or pharmaceutically acceptable salts forms thereof.

A further aspect of the present invention includes compounds of formula (IV)
wherein Y is selected from:
a) C₁-C₄ alkylene-R¹;
b) C₁-C₄ alkylene-R²;
c) C₁-C₄ alkylene-O(CH₂)ₚOR²¹,
d) CH₂C(OH)(CH₃)₂, CH₂C(CH₃)₂OH, CH₂C(OH)₂CF₃, CH₂C(OH)(C≡CH)₂, or CH₂CH(OH)CH₃,
e) CH₂CH=CH₂, or CH₂C(=C)CH₃ except when X is a bond and q is 2;
wherein said alkylene groups are optionally substituted with an R²⁰ group;
R¹ is selected from pyrrolidinyl, piperidinyl, morpholinyl, NR²¹C(=O)R¹⁴, C(=O)R¹⁵, CO₂R¹¹, C(=O)NR¹²R¹³, C(=O)NR²¹OR¹⁴, NR²¹S(O)₂R¹¹, S(O)₂NR^{12A}R^{13A}, and PO(OR²¹)₂;
R² is furyl, thienyl, or triazolonyl;
wherein said R² groups are optionally substituted with an R²⁰ group;
R¹¹ at each occurrence is independently C₁-C₄ alkyl;
R¹² and R¹³ at each occurrence are each independently selected from H and C₁-C₄ alkyl, optionally substituted with C(=O)NR^{12A}R^{13A}, or R¹² and R¹³, together with the nitrogen to which they are attached, form a pyrrolidinyl or piperidinyl ring;
R^{12A} and R^{13A} at each occurrence are each independently selected from H and C₁-C₄ alkyl;
R¹⁴ at each occurrence is independently C₁-C₄ alkyl;
R¹⁵ at each occurrence is independently selected from C₁-C₄ alkyl, and thienyl;
R²⁰ at each occurrence is independently selected from F, Cl, Br, I, OR²¹, OR²⁵, NR²³R²⁴, NHOH, NO₂, CN, CF₃, =O, C(=O)R²², CO₂R²¹, C(=O)NR²³R²⁴, or NR²¹C(=O)R²²;
R²¹ at each occurrence is independently selected from H and C₁-C₄ alkyl;
R²² at each occurrence is independently C₁-C₄ alkyl;
R²³ and R²⁴ at each occurrence are each independently selected from H and C₁-C₄ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
p is 1, 2, 3, or 4;
q is 0, 1, or 2.

In a further embodiment of the present invention there are provided compounds of formula (V): 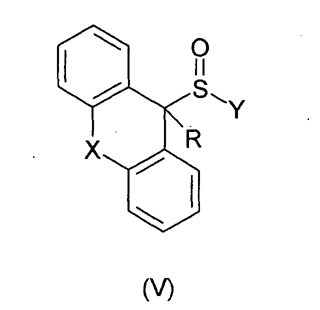 wherein
the phenylene rings are each independently optionally substituted with one to three R²⁰ groups;
X is a bond, O, S(O)_{y}, NR¹⁰, C₂ alkylene, or C₂ alkenylene, wherein said alkylene and alkenylene groups are optionally substituted with an R²⁰ group;
R is H or C₁-C₄ alkyl;
Y is selected from:
a) C₁-C₆ alkylene-R¹;
b) CH₂CR²¹=C(R²¹)₂ CH₂CR²¹=C(R²¹)₂ except when X is a bond and q is 2;
R¹ is selected from pyrrolidinyl, piperidinyl, morpholinyl, NR²¹C(=O)R¹⁴, C(=O)NR¹²R¹³, and NR²¹S(O)₂R¹¹;
R¹⁰ is independently selected from H, C₁-C₆ alkyl, C₆-C₁₀ aryl, C(=O)R¹⁴, and S(O)_{y}R¹⁴;
wherein said alkyl and aryl groups are optionally substituted with one to three R²⁰ groups;
R¹¹ at each occurrence is independently C₁-C₆ alkyl;
R¹² and R¹³ at each occurrence are each independently selected from H and C₁-C₆ alkyl, or R¹² and R¹³, together with the nitrogen to which they are attached, form a 5-6 membered heterocycloalkyl;
wherein said alkyl and heterocycloalkyl groups are optionally substituted with an R²⁰ group;
R¹⁴ at each occurrence is independently C₁-C₆ alkyl;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and phenyl;
R²³ and R²⁴ at each occurrence are each independently selected from H and C₁-C₆ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
y is 0, 1, or 2;
with the following proviso:
1) when R = H and Y is (C₁-C₆ alkylene)-C(=O)NR¹²R¹³, then the alkylene group must be substituted with a spirocycloalkyl group;
and the stereoisomeric forms, mixtures of stereoisomeric forms or pharmaceutically acceptable salts forms thereof.

A further aspect of the present invention includes compounds of formula (V) wherein X is a bond; R is C₁-C₄ alkyl, Y is C₁-C₆ alkyl-C(=O)NR¹²R¹³.

Another aspect includes compounds of formula (V) wherein X is a bond; R is H, Y is C₁-C₆ alkyl-R¹, and R¹ is selected from pyrrolidinyl, piperidinyl, morpholinyl, NR²¹C(=O)R¹⁴, or NR²¹S(O)₂R¹¹.

A further aspect includes compounds of formula (V) wherein X is a bond and Y is CH₂CR²¹=C(R²¹)₂ CH₂CR²¹=C(R²¹)₂.

In yet another aspect, there are included compounds of formula (V) wherein Y is CH₂CH=CH₂, or CH₂C(=C)CH₃.

An additional aspect includes compounds of formula (V) wherein the phenylene rings are each independently optionally substituted with one to three R²⁰ groups;
q is 1;
X is a bond;
Y is selected from:
a) C₁-C₄ alkylene-R¹;
b) CH₂CH=CH₂, or CH₂C(=C)CH₃ except when X is a bond and q is 2;
   R¹ is selected from pyrrolidinyl, piperidinyl, morpholinyl, NR²¹C(=O)R¹⁴, C(=O)NR¹²R¹³, and NR²¹S(O)₂R¹¹;
   R¹¹ at each occurrence is independently C₁-C₄ alkyl;
   R¹² and R¹³ at each occurrence are each independently selected from H and C₁-C₄ alkyl, optionally substituted with C(=O)NR^{12A}R^{13A}, or R¹² and R¹³, together with the nitrogen to which they are attached, form a pyrrolidinyl or piperidinyl ring;
   R^{12A} and R^{13A} at each occurrence are each independently selected from H and C₁-C₄ alkyl; R¹⁴ at each occurrence is independently C₁-C₄ alkyl;
   R²⁰ at each occurrence is independently selected from F, Cl, Br, I, OR²¹, OR²⁵, NR²³R²⁴, NHOH, NO₂, CN, CF₃, =O, C(=O)R²², CO₂R²¹, C(=O)NR²³R²⁴, or NR²¹C(=O)R²²;
   R²¹ at each occurrence is independently selected from H and C₁-C₄ alkyl;
   R²² at each occurrence is independently C₁-C₄ alkyl;
   R²³ and R²⁴ at each occurrence are each independently selected from H and C₁-C₄ alkyl, or R²³ and R²⁴, together with the nitrogen to which they are attached, form a 5-6 membered heterocycloalkyl;
   R²⁵ at each occurrence is independently the residue of an amino acid after the hydroxyl group of the carboxyl group is removed.

In yet another embodiment of the present invention there are provided compounds of formula (VI): 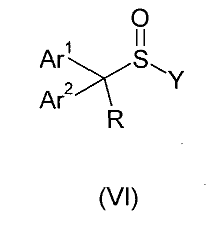 wherein
Ar¹ and Ar² are each independently phenyl optionally substituted with one to three R²⁰ groups;
R is H or C₁-C₄ alkyl;
Y is selected from:
a) C₁-C₆ alkylene-R¹;
b) C₁-C₆ alkylene-R²;
c) C₁-C₆ alkylene-O(CH₂)ₚOR²¹,
d) CH₂C(OH)(CH₃)₂, CH₂C(CH₃)₂OH, CH₂C(OH)₂CF₃, CH₂C(OH)(C≡CH)₂, or CH₂CH(OH)CH₃;
R¹ is selected from C(=O)R¹⁵, CO₂R¹¹, C(=O)NR¹²R¹³, C(=O)NR²¹OR¹⁴, S(O)₂NR^{12A}R^{13A}, and PO(OR²¹)₂;
R² is furyl, thienyl, or triazolyl; wherein said R² groups are optionally substituted with an R²⁰ group;
R¹¹ at each occurrence is independently C₁-C₆ alkyl;
R¹² and R¹³ at each occurrence are each independently selected from H and C₁-C₆ alkyl, or R¹² and R¹³, together with the nitrogen to which they are attached, form a 5-6 membered heterocycloalkyl;
R^{12A} and R^{13A} at each occurrence are each independently selected from H and C₁-C₆ alkyl; R¹⁴ at each occurrence is independently C₁-C₆ alkyl;
R¹⁵ at each occurrence is independently selected from C₁-C₆ alkyl, and 5-membered heteroaryl;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl; and phenyl;
R²³ and R²⁴ at each occurrence are each independently selected from H and C₁-C₆ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
pis 1, 2, 3, or 4;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group; and the stereoisomeric forms, mixtures of stereoisomeric forms or pharmaceutically acceptable salts forms thereof.

A further aspect of the present invention includes compounds of formula (VI) wherein R is C₁-C₄ alkyl, and Y is C₁-C₆ alkyl-C(=O)NR¹²R¹³.

Another aspect includes compounds of formula (VI) wherein R is H, and Y is C₁-C₆ alkyl-R¹, wherein said alkyl is substituted with spirocycloalkyl, and R¹ is CO₂R¹¹, or C(=O)NR¹²R¹³.

A further aspect includes compounds of formula (VI) wherein R¹ is C(=O)NR¹²R¹³.

In yet another aspect, there are included compounds of formula (VI) wherein R¹ is selected from C(=O)R¹⁵, C(=O)NR²¹OR¹⁴, S(O)₂NR^{12A}R^{13A}, and PO(OR²¹)₂.

An additional aspect includes compounds of formula (VI) wherein Y is C₁-C₆ alkylene-O(CH₂)ₚOR²¹.

In a further aspect, there are compounds of formula (VI) wherein Y is CH₂C(OH)(CH₃)₂, CH₂C(CH₃)₂OH, CH₂C(OH)₂CF₃, CH₂C(OH)(C≡CH)₂, or CH₂CH(OH)CH₃.

In an additional aspect, there are compounds of formula (VI) wherein Ar¹ and Ar² are each independently phenyl optionally substituted with one to three R²⁰ groups;
R is H or C₁-C₄ alkyl;
Y is selected from:
a) C₁-C₄ alkylene-R¹;
b) C₁-C₄ alkylene-R²;
c) CH₂CH₂O(CH₂)₂OCH₃,
d) CH₂C(OH)(CH₃)₂, CH₂C(CH₃)₂OH, CH₂C(OH)₂CF₃, CH₂C(OH)(C≡CH)₂, or CH₂CH(OH)CH₃,
R¹ is selected from C(=O)R¹⁵, CO₂R¹¹, C(=O)NR¹²R¹³, C(=O)NR²¹OR¹⁴, S(O)₂NR^{12A}R^{13A}, and PO(OR²¹)₂;
R² is furyl, thienyl, or triazolonyl;
R¹¹ at each occurrence is independently C₁-C₄ alkyl;
R¹² and R¹³ at each occurrence are each independently selected from H and C₁-C₄ alkyl;
R^{12A} and R^{13A} at each occurrence are each independently selected from H and C ₁-C₄ alkyl;
R¹⁴ at each occurrence is independently C₁-C₄ alkyl;
R¹⁵ at each occurrence is independently selected from C₁-C₄ alkyl, and thienyl;
R²⁰ at each occurrence is independently selected from F, Cl, Br, I, OR²¹, OR²⁵, NR²³R²⁴, NHOH, NO₂, CN, CF₃, C₃-C₆ spirocycloalkyl, =O, C(=O)R²², CO₂R²¹, C(=O)NR²³R²⁴, or NR²¹C(=O)R²²;
R²¹ at each occurrence is independently selected from H and C₁-C₄ alkyl;
R²² at each occurrence is independently C₁-C₄ alkyl;
R²³ and R²⁴ at each occurrence are each independently selected from H and C₁-C₄ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
with the following proviso:
1) when R = H, Y is (C₁-C₄ alkylene)-R¹, and R¹ is CO₂R¹¹ or C(=O)NR¹²R¹³, then the alkylene group must be substituted with a spirocycloalkyl group.

In still more aspects, there are compounds of formula (VI) wherein R is H; or R¹ selected from C(=O)R¹⁵, CO₂R¹¹, C(=O)NR¹²R¹³, C(=O)NR²¹OR¹⁴, S(O)₂NR^{12A}R^{13A}, and PO(OR²¹)₂; or Y is C₁-C₄ alkylene-R¹.

In additional aspects of the present invention there are included compounds of any of the preceding formulas wherein q is 1 or 2. In certain aspects, q is 0. In other aspects q is 1. In further aspects, q is 2.

In other aspects of the present invention, there are included compounds of any of the preceding formulas wherein q can be any moieties of the previous embodiments, and R can be selected as follows. In one aspect, R is H. In other aspects, R is C₁-C₆ alkyl. In additional aspects, R is C₁-C₄ alkyl, preferably methyl or ethyl, and more preferably methyl.

In certain aspects of the present invention, there are included compounds of any of the preceding formulas wherein q and R can be any moieties of the previous embodiments, and rings A and B can be selected as follows. In one aspect, rings A and B are phenylene. In other aspects, rings A and B are each independently selected from: a) a 6-membered aromatic carbocyclic ring in which from 1 to 3 carbon atoms may be replaced by nitrogen atoms, preferably pyridylene, pyrazinylene, or pyrimidinylene; and b) a 5-membered aromatic carbocyclic ring in which either: i) one carbon atom may be replaced with an oxygen, nitrogen, or sulfur atom; ii) two carbon atoms may be replaced with a sulfur and a nitrogen atom, an oxygen and a nitrogen atom, or two nitrogen atoms; or iii) three carbon atoms may be replaced with three nitrogen atoms, one oxygen and two nitrogen atoms, or one sulfur and two nitrogen atoms. In an additional aspect, rings A and B are each independently selected from phenylene, pyridylene, thienylene, or a 5-membered aromatic ring in which one or two carbon atoms may be replaced with a nitrogen atom. In a further aspect, rings A and B are each independently selected from phenylene, pyridylene, pyrazinylene, pyrimidinylene, pyrrolylene, pyrazolylene, imidazolylene, furylene, and thienylene.

In another aspect of the present invention, there are included compounds of any of the preceding formulas wherein q, R, and rings A and B can be any moieties of the previous embodiments, and X can be selected as follows. In one aspect, X is not present, is a bond, O, C₂ alkylene, or C(=O). In a further aspect, X is not present or is a bond. In another aspect, X is not present, and preferably the A-B-X moiety is Ph₂CH. In an additional aspect, X is a bond, and preferably the tricyclic A-B-X moiety is fluorenyl. In another aspect, X is O, S(O)_{y}, NR¹⁰, and preferably O. Another aspect includes X as C₂ alkylene. In a further aspect, X is C₂₋₃ alkenylene, C(=O), C(R²¹)₂NR¹⁰, C(R²¹)=N, N=C(R²¹), C(=O)N(R¹⁰), or NR¹⁰C(=O).

In certain aspects of the present invention, there are included compounds of any of the preceding formulas wherein q, R, rings A and B, X and Y can be any moieties of the previous embodiments, and Y is C₁-C₆ alkylene-R¹, particularly those where Y is C₁-C₄ alkylene-R¹, or Y is CH₂-R¹ or Y is CH₂CH₂-R¹.

Other aspects of the present invention include compounds of any of the preceding formulas wherein q, R, rings A and B, and X can be any moieties of the previous embodiments, and Y can be selected as follows. One aspect is where Y is C₁-C₆ alkylene-R², particularly those where R² is furyl, thienyl or triazinyl, or 2-triazolonyl. In another aspect, Y is C₁-C₆ alkylene-O(CH₂)ₚOR²¹, particularly those where Y is CH₂CH₂O(CH₂)₂OCH₃. In a further aspect, Y is C₁-C₆ alkyl substituted with one or two OR¹¹ groups, wherein said alkyl group is further optionally substituted with 1-3 R²⁰ groups, and in particular Y is CH₂C(OH)(CH₃)₂, or CH₂C(CH₃)₂OH, or CH₂C(OH)₂CF₃, or CH₂C(OH)(C≡CH)₂, or CH₂CH(OH)CH₃. In an additional aspect, Y is CH₂CR²¹=C(R²¹)₂, and in particular Y is CH₂CH=CH₂, or CH₂C(=C)CH₃.

Additional aspects of the present invention include compounds of any of the preceding formulas wherein q, R, rings A and B, X and Y can be any moieties of the previous embodiments, and Y is (C₁-C₄ alkylene)ₘ-Z¹-(C₁-C₄ alkylene)ₙ-R¹, particularly those where Y is C₁-C₄ alkylene-Z¹-R¹, or Y is Z¹-C₁-C₄ alkylene-R¹, or Y is C₁-C₄ alkylene-Z¹-C₁-C₄ alkylene-R¹.

Further aspects of the present invention include compounds of any of the preceding formulas wherein q, R, rings A and B, X, and Y can be any moieties of the previous embodiments, and Z¹ can be selected as follows. In one aspect, Z¹ is CR²¹=CR²¹, C=C(R²¹), C=C, or phenylene, or more particularly where Z¹ is CR²¹=CR²¹ or Z¹ is phenylene. Other aspects include compounds where Z¹ is CR²¹=CR²¹, or C=C. Other aspects include compounds where Z¹ is C₃-C₆ cycloalkylene, and in particular, cyclopentylene or cyclohexylene. Other aspects include compounds where Z¹ is 5-10 membered heteroarylene, in particular 5-6 membered heteroarylenes containing nitrogen, preferably containing 1 or 2 nitrogen atoms. Additional aspects include compounds where Z¹ is 3-6 membered heterocycloalkylene.

Further aspects of the present invention include compounds of any of the preceding formulas wherein q, R, rings A and B, X and Y can be any moieties of the previous embodiments, and Y is C₁-C₄ alkylene-Z²-C₁-C₄ alkylene or Y is C₁-C₄ alkylene-Z², wherein Z² is O, NR^{10A}, or S(O)_{y}, particularly those where Z² is O. Additional aspects include any of the above embodiments of Y wherein Z² is NR^{10A}.

Further aspects of the present invention include compounds of any of the preceding formulas wherein q, R, rings A and B, X, Y, Z¹, and Z² can be any moieties of the previous embodiments, and R¹ can be any moiety selected from the following enumerated paragraphs:
1. NR¹²R¹³, particularly those wherein R¹² and R¹³ at each occurrence are each independently selected from H and C₁-C₆ alkyl, or those where R¹² and R¹³, together with the nitrogen to which they are attached, form a 5-6 membered heterocycloalkyl, particularly pyrrolidinyl, piperidinyl, or morpholinyl.
2. NR²¹C(=O)R¹⁴.
3. C(=O)R¹⁵, particularly those where R¹⁵ is C₁-C₄ alkyl, or thienyl.
4. CO₂R¹¹, particularly those where R¹¹ is C₁-C₄ alkyl.
5. OC(=O)R¹¹.
6. C(=O)NR¹²R¹³.
7. C(=O)NR²¹OR¹⁴.
8. C(=NR¹¹)NR¹²R¹³.
9. NR²¹S(O)₂R¹¹.
10. S(O)₂NR¹²R¹³.
11. NR²¹S(O)₂NR¹²R¹³.
12. PO(OR²¹)₂.

Other additional aspects of the present invention include compounds of any of the preceding formulas wherein q, R, rings A and B, X, Y, Z¹, and Z² can be any moieties of the previous embodiments, and R¹ can be a combination of the values selected from the previous enumerated paragraphs. The preceding enumerated paragraphs may be combined to further define additional preferred embodiments of compounds of any of the preceding formulas. For example, one such combination includes NR¹²R¹³, NR²¹C(=O)R¹⁴, C(=O)R¹⁵, CO₂R¹¹, OC(=O)R¹¹, C(=O)NR¹²R¹³, C(=NR¹¹)NR¹²R¹³, NR²¹S(O)₂R¹¹, S(O)₂NR¹²R¹³, NR²¹S(O)₂NR¹²R¹³, or PO(OR²¹)₂. An additional combination includes NR¹²R¹³, NR²¹C(=O)R¹⁴, C(=O)NR¹²R¹³, C(=NR¹¹)NR¹²R¹³, NR²¹S(O)₂R¹¹, S(O)₂NR¹²R¹³, NR²¹S(O)₂NR¹²R¹³, or PO(OR²¹)₂.

A third such combination includes NR¹²R¹³, NR²¹C(=O)R¹⁴, C(=O)R¹⁵, C(=O)NR¹²R¹³, C(=O)NR²¹OR¹⁴, NR²¹S(O)₂R¹¹, S(O)₂NR¹²R¹³, and PO(OR²¹)₂.

A fourth such combination includes NR¹²R¹³, NR²¹C(=O)R¹⁴, C(=O)NR¹²R¹³, NR²¹S(O)₂R¹¹, or S(O)₂NR¹²R¹³.

A fifth such combination includes NR¹²R¹³, NR²¹C(=O)R¹⁴, C(=O)R¹⁵, C(=O)NR²¹OR¹⁴, NR²¹S(O)₂R¹¹, S(O)₂NR¹²R¹³, and PO(OR²¹)₂.

A sixth such combination includes C(=O)R¹⁵, C(=O)NR²¹OR¹⁴, S(O)₂NR^{12A}R^{13A}, and PO(OR²¹)₂.

A seventh such combination includes C(=O)R¹⁵, C(=O)NR¹²R¹³, C(=O)NR²¹OR¹⁴, S(O)₂NR^{12A}R^{13A}, and PO(OR²¹)₂.

An eighth such combination includes NR²¹C(=O)R¹⁴, C(=O)NR¹²R¹³, NR²¹S(O)₂R¹¹, and S(O)₂NR¹²R¹³.

Further aspects of the present invention include compounds of any of the preceding formulas wherein q, R, rings A and B, X, Y, Z¹, Z², and R¹ can be any moieties of the previous embodiments, and R¹² and R¹³ are independently H or C₁-C₆ alkyl, or where R¹² and R¹³, together with the nitrogen to which they are attached, form a 3-7 membered heterocycloalkyl ring. In another aspects, R¹² and R¹³, together with the nitrogen to which they are attached, form a 5-6 membered heterocycloalkyl ring containing from 1 to 2 nitrogen atoms, or more preferably form pyrrolidinyl, piperidinyl, or morpholinyl. In certain aspects the heterocycloalkyl rings can be substituted with one R²⁰ group, and in other aspects, the heterocycloalkyl rings are unsubstituted.

The following terms and expressions contained herein are defined as follows:

As used herein, the term "about" refers to a range of values from ± 10% of a specified value. For example, the phrase "about 50 mg" includes ± 10% of 50, or from 45 to 55 mg.

As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein "stable compound" or "stable structure" refers to a compound that is sufficiently robust to survive isolation to a useful degree of purity from a reaction mixture, and preferably capable of formulation into an efficacious therapeutic agent. The present invention is directed only to stable compounds.

As used herein, the term "alkyl" refers to a straight-chain, or branched alkyl group having 1 to 8 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, octyl, etc. The alkyl moiety of alkyl-containing groups, such as alkoxy, alkoxycarbonyl, and alkylaminocarbonyl groups, has the same meaning as alkyl defined above. Lower alkyl groups, which are preferred, are alkyl groups as defined above which contain 1 to 4 carbons. A designation such as "C₁-C₄ alkyl" refers to an alkyl radical containing from 1 to 4 carbon atoms.

As used herein, the term "alkenyl" refers to a straight chain, or branched hydrocarbon chains of 2 to 8 carbon atoms having at least one carbon-carbon double bond. A designation "C₂-C₈ alkenyl" refers to an alkenyl radical containing from 2 to 8 carbon atoms. Examples of alkenyl groups include ethenyl, propenyl, isopropenyl, 2,4-pentadienyl, etc.

As used herein, the term "alkynyl" refers to a straight chain, or branched hydrocarbon chains of 2 to 8 carbon atoms having at least one carbon-carbon triple bond. A designation "C₂-C₈ alkynyl" refers to an alkynyl radical containing from 2 to 8 carbon atoms. Examples include ethynyl, propynyl, isopropynyl, 3,5-hexadiynyl, etc.

As used herein, the term "alkylene" refers to a substituted or unsubstituted, branched or straight chained hydrocarbon of 1 to 8 carbon atoms, which is formed by the removal of two hydrogen atoms. A designation such as "C₁-C₄ alkylene" refers to an alkylene radical containing from 1 to 4 carbon atoms. Examples include methylene (-CH₂-), propylidene (CH₃CH₂CH=), 1,2-ethandiyl (-CH₂CH₂-), etc.

As used herein, the term "phenylene" refers to a phenyl group with an additional hydrogen atom removed, i.e. a moiety with the structure of:

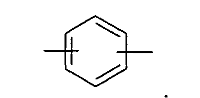

As used herein, the terms "carbocycle", "carbocyclic" or "carbocyclyl" refer to a substituted or unsubstituted, stable monocyclic or bicyclic hydrocarbon ring system which is saturated, partially saturated or unsaturated, and contains from 3 to 10 ring carbon atoms. Accordingly the carbocyclic group may be aromatic or non-aromatic, and includes the cycloalkyl and aryl compounds defined herein. The bonds connecting the endocyclic carbon atoms of a carbocyclic group may be single, double, triple, or part of a fused aromatic moiety.

As used herein, the term "cycloalkyl" refers to a saturated or partially saturated mono- or bicyclic alkyl ring system containing 3 to 10 carbon atoms. A designation such as "C₅-C₇ cycloalkyl" refers to a cycloalkyl radical containing from 5 to 7 ring carbon atoms. Preferred cycloalkyl groups include those containing 5 or 6 ring carbon atoms. Examples of cycloalkyl groups include such groups as cyclopropyl, cyclobutyl, cyclopentyl, cyclohexl, cycloheptyl, cyclooctyl, pinenyl, and adamantanyl.

As used herein, the term "aryl" refers to a substituted or unsubstituted, mono- or bicyclic hydrocarbon aromatic ring system having 6 to 12 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

As used herein, the terms "heterocycle", "heterocyclic" or "heterocyclyl" refer to a substituted or unsubstituted carbocyclic group in which the ring portion includes at least one heteroatom such as O, N, or S. The nitrogen and sulfur heteroatoms may be optionally oxidized, and the nitrogen may be optionally substituted in non-aromatic rings. Heterocycles are intended to include heteroaryl and heterocycloalkyl groups.

As used herein, the term "heterocycloalkyl" refers to a cycloalkyl group in which one or more ring carbon atoms are replaced by at least one hetero atom such as -O-, -N-, or -S-. Examples of heterocycloalkyl groups include pyrrolidinyl, pyrrolinyl, imidazolidinyl, imidazolinyl, pyrazolidinyl, pyrazolinyl, pyrazalinyl, piperidyl, piperazinyl, morpholinyl, thiomorpholinyl, tetrahydrofuranyl, dithiolyl, oxathiolyl, dioxazolyl, oxathiazolyl, pyranyl, oxazinyl, oxathiazinyl, and oxadiazinyl.

As used herein, the term "heteroaryl" refers to an aromatic group containing 5 to 10 ring carbon atoms in which one or more ring carbon atoms are replaced by at least one hetero atom such as -O-, -N-, or -S-. Examples of heteroaryl groups include pyrrolyl, furyl, thienyl, pyrazolyl, imidazolyl, thiazolyl, isothiazolyl, isoxazolyl, oxazolyl, oxathiolyl, oxadiazolyl, triazolyl (including 1,2,3 triazolyl, 1,2,4 triazolyl, and 3-oxo-1,2,4 triazolyl), oxatriazolyl, furazanyl, tetrazolyl, pyridyl, pyrazinyl, pyrimidinyl, pyridazinyl, triazinyl, picolinyl, indolyl, isoindolyl, indazolyl, benzofuranyl, isobenzofuranyl, purinyl, quinazolinyl, quinolyl, isoquinolyl, benzoimidazolyl, benzothiazolyl, benzothiophenyl, thianaphthenyl, benzoxazolyl, benzisoxazolyl, cinnolinyl, phthalazinyl, naphthyridinyl, and quinoxalinyl. Included within the definition of "heteroaryl" are fused ring systems, including, for example, ring systems in which an aromatic ring is fused to a heterocycloalkyl ring. Examples of such fused ring systems include, for example, phthalamide, phthalic anhydride, indoline, isoindoline, tetrahydroisoquinoline, chroman, isochroman, chromene, and isochromene.

As used herein, the term "arylalkyl" refers to an alkyl group that is substituted with an aryl group. Examples of arylalkyl groups include, but are not limited to, benzyl, bromobenzyl, phenethyl, benzhydryl, diphenylmethyl, triphenylmethyl, diphenylethyl, naphthylmethyl, etc.

As used herein, the term "spirocycloalkyl" refers to a cycloalkyl group bonded to a carbon chain or carbon ring moiety by a carbon atom common to the cycloalkyl group and the carbon chain or carbon ring moiety. For example, a C₃ alkyl group substituted with an R group wherein the R group is spirocycloalkyl containing 5 carbon atoms refers to:

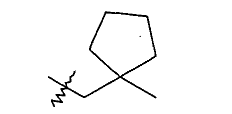

As used herein, the term "amino acid" refers to a group containing both an amino group and a carboxyl group. Embodiments of amino acids include α-amino, β-amino, γ-amino acids. The α-amino acids have a general formula HOOC-CH(side chain)-NH₂. In certain embodiments, substituent groups for the compounds of the present invention include the residue of an amino acid after removal of the hydroxyl moiety of the carboxyl group thereof; i.e., groups of formula -C(=O)CH(NH₂)-(side chain). The amino acids can be in their D, L or racemic configurations. Amino acids include naturally-occurring and non-naturally occurring moieties. The naturally-occurring amino acids include the standard 20 α-amino acids found in proteins, such as glycine, serine, tyrosine, proline, histidine, glutamine, etc. Naturally-occurring amino acids can also include non-α-amino acids (such as β-alanine, γ-aminobutyric acid, homocysteine, etc.), rare amino acids (such as 4-hydroxyproline, 5-hydroxylysine, 3-methylhistidine, etc.) and non-protein amino acids (such as citrulline, ornithine, canavanine, etc.). Non-naturally occurring amino acids are well-known in the art, and include analogs of natural amino acids. See Lehninger, A. L. *Biochemistry,* 2^{nd} ed.; Worth Publishers: New York, **1975,** pp. 71-77, the disclosure of which is incorporated herein by reference. Non-naturally occurring amino acids also include α-amino acids wherein the side chains are replaced with synthetic derivatives. Representative side chains of naturally occurring and non-naturally occurring α-amino acids are shown below in Table A.

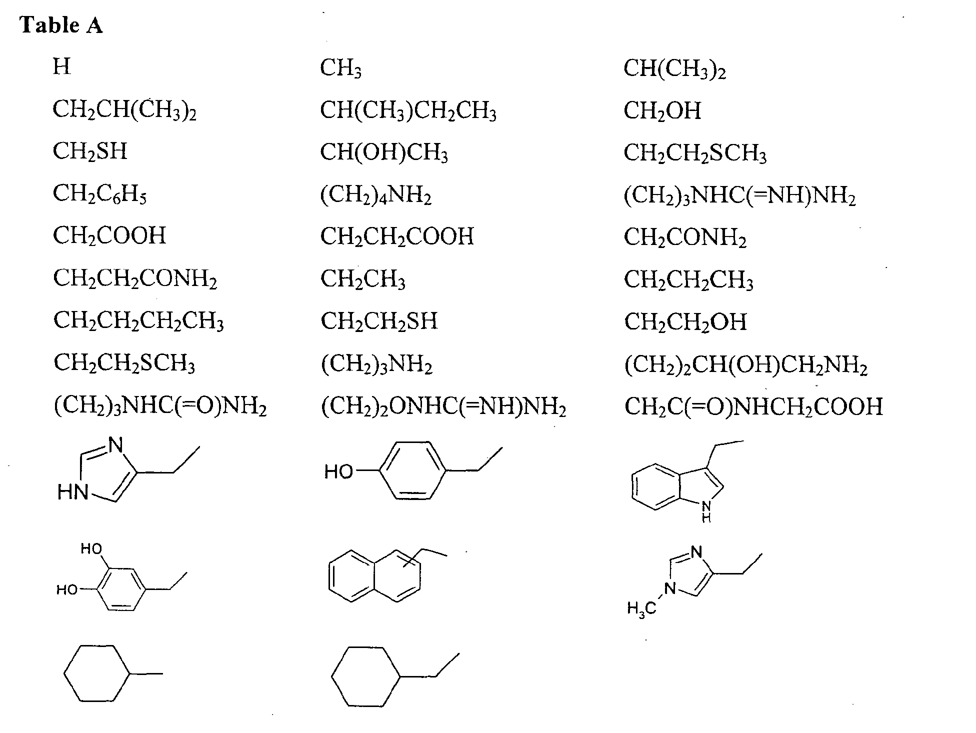

As used herein, the term "subject" refers to a warm blooded animal such as a mammal, preferably a human, or a human child, which is afflicted with, or has the potential to be afflicted with one or more diseases and conditions described herein.

As used herein, a "therapeutically effective amount" refers to an amount of a compound of the present invention effective to prevent or treat the symptoms of particular disorder. Such disorders include, but are not limited to, those pathological and neurological disorders associated with the aberrant activity described herein, wherein the treatment or prevention comprises inhibiting, inducing, or enhancing the activity thereof by contacting the receptor with a compound of the present invention.

As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, the term "unit dose" refers to a single dose which is capable of being administered to a patient, and which can be readily handled and packaged, remaining as a physically and chemically stable unit dose comprising either the active compound itself, or as a pharmaceutically acceptable composition, as described hereinafter.

All other terms used in the description of the present invention have their meanings as is well known in the art.

In another aspect, the present invention is directed to pharmaceutically acceptable salts of the compounds described above. As used herein, "pharmaceutically acceptable salts" includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.

Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

In addition to pharmaceutically-acceptable salts, other salts are included in the invention. They may serve as intermediates in the purification of the compounds, in the preparation of other salts, or in the identification and characterization of the compounds or intermediates.

The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

The present invention also encompasses the pharmaceutically acceptable prodrugs of the compounds disclosed herein. As used herein, "prodrug" is intended to include any compounds which are converted by metabolic processes within the body of a subject to an active agent that has a formula within the scope of the present invention. Since prodrugs are known to enhance numerous desirable qualities of pharmaceuticals (e.g., solubility, bioavailability, manufacturing, etc.) the compounds of the present invention may be delivered in prodrug form. Conventional procedures for the selection and preparation of suitable prodrug derivatives are described, for example, in *Prodrugs,* Sloane, K. B., Ed.; Marcel Dekker: New York, 1992, which is incorporated herein by reference in its entirety.

It is recognized that compounds of the present invention may exist in various stereoisomeric forms. As such, the compounds of the present invention include all stereoisomeric forms, such as the diastereomeric and enantiomeric forms. The compounds are normally prepared as racemates and can conveniently be used as such, but individual stereoisomers can be isolated or synthesized by conventional techniques if so desired. Such stereoisomeric forms are included in the present invention, including the racemates, individual enantiomers and diastereomers, and mixtures thereof.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. *Stereochemistry of Organic Compounds;* Wiley: New York, 1994, and Jacques, J, et al. *Enantiomers, Racemates, and Resolutions;* Wiley: New York, 1981, each incorporated by reference herein in their entireties.

It is further recognized that functional groups present on the compounds of the present invention may contain protecting groups. For example, the amino acid side chain substituents of the compounds of the present invention can be substituted with protecting groups such as benzyloxycarbonyl or t-butoxycarbonyl groups. Protecting groups are known per se as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Preferred groups for protecting lactams include silyl groups such as t-butyldimethylsilyl ("TBDMS"), dimethoxybenzhydryl ("DMB"), acyl, benzyl ("Bn"), and methoxybenzyl groups. Preferred groups for protecting hydroxy groups include TBS, acyl, benzyl, benzyloxycarbonyl ("CBZ"), t-butyloxycarbonyl ("Boc"), and methoxymethyl. Many other standard protecting groups employed by one skilled in the art can be found in Greene, T.W. and Wuts, P.G.M., "Protective Groups in Organic Synthesis" 2d. Ed., Wiley & Sons, 1991.

### Synthesis and Examples

The compounds of the present invention may be prepared in a number of methods well known to those skilled in the art, including, but not limited to those described below, or through modifications of these methods by applying standard techniques known to those skilled in the art of organic synthesis. The reagents and starting materials are commercially available, or readily synthesized by well-known techniques by one of ordinary skill in the arts. All substituents in the synthetic schemes, unless otherwise indicated, are as previously defined. All processes disclosed in association with the present invention are contemplated to be practiced on any scale, including milligram, gram, multigram, kilogram, multikilogram or commercial industrial scale.

Illustrative of compounds encompassed by the present invention that are useful in the utilities disclosed herein include those set forth in the following tables. This list is meant to be representative only and is not intended to limit the scope of the invention in any way.

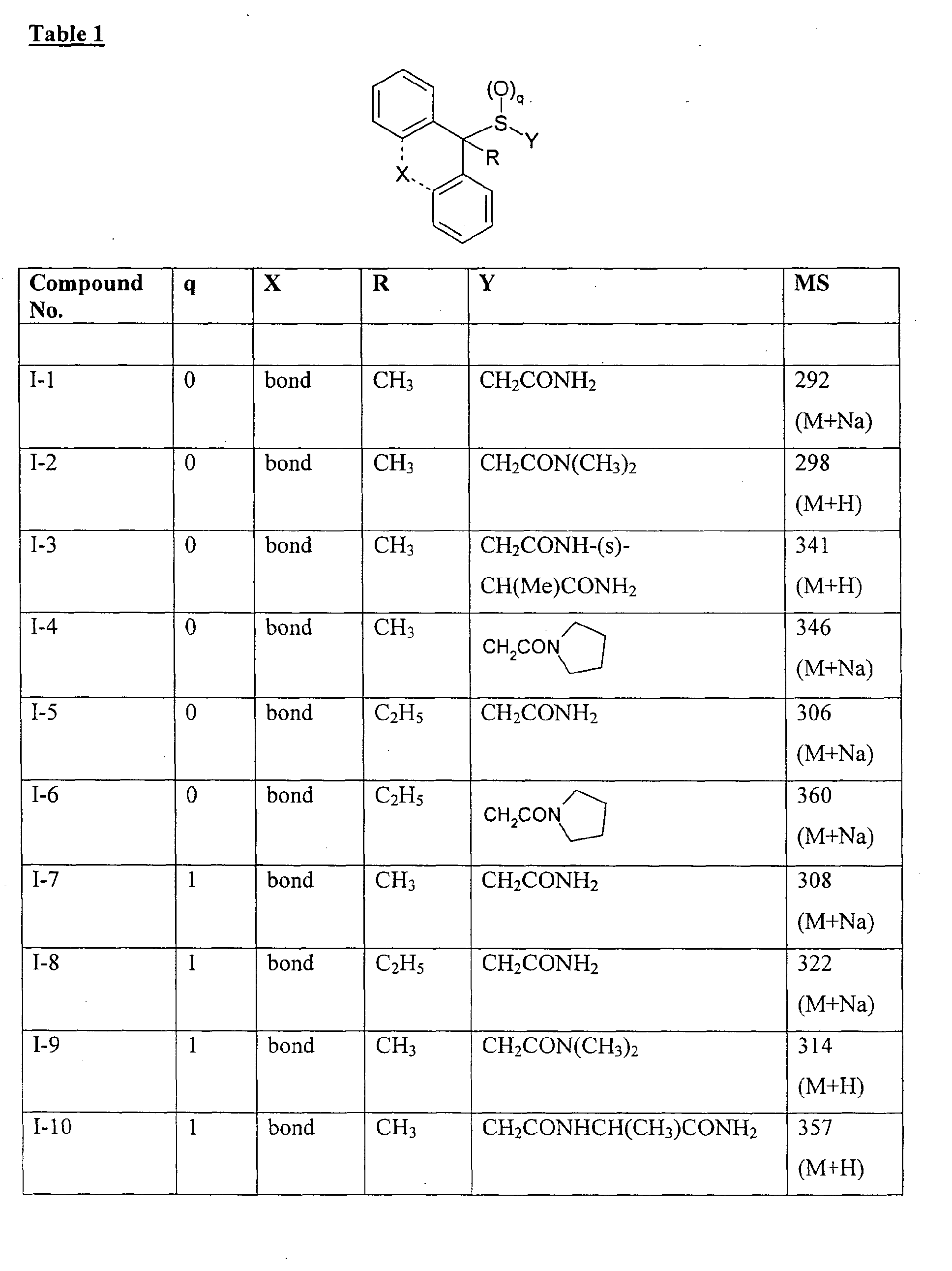

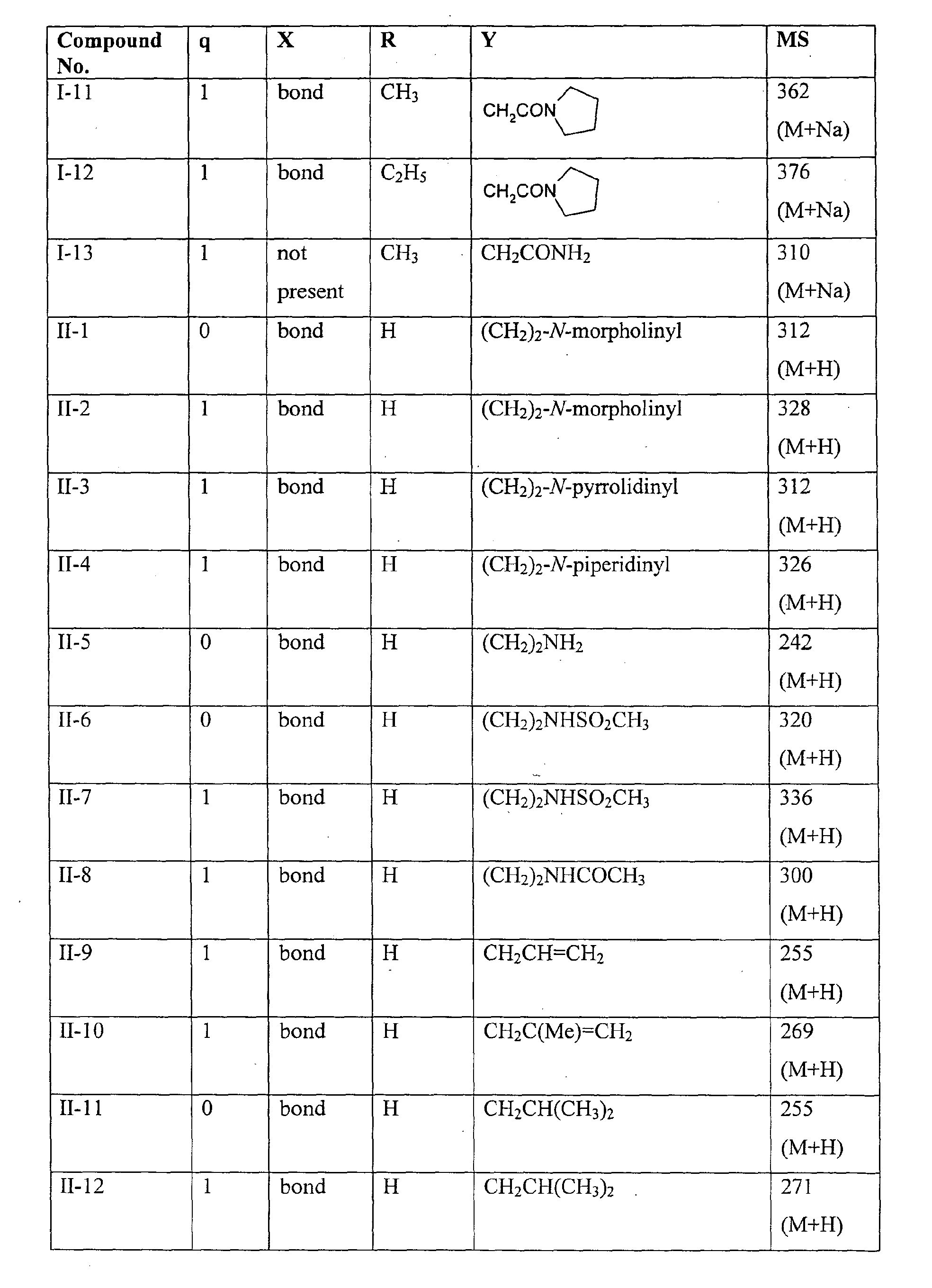

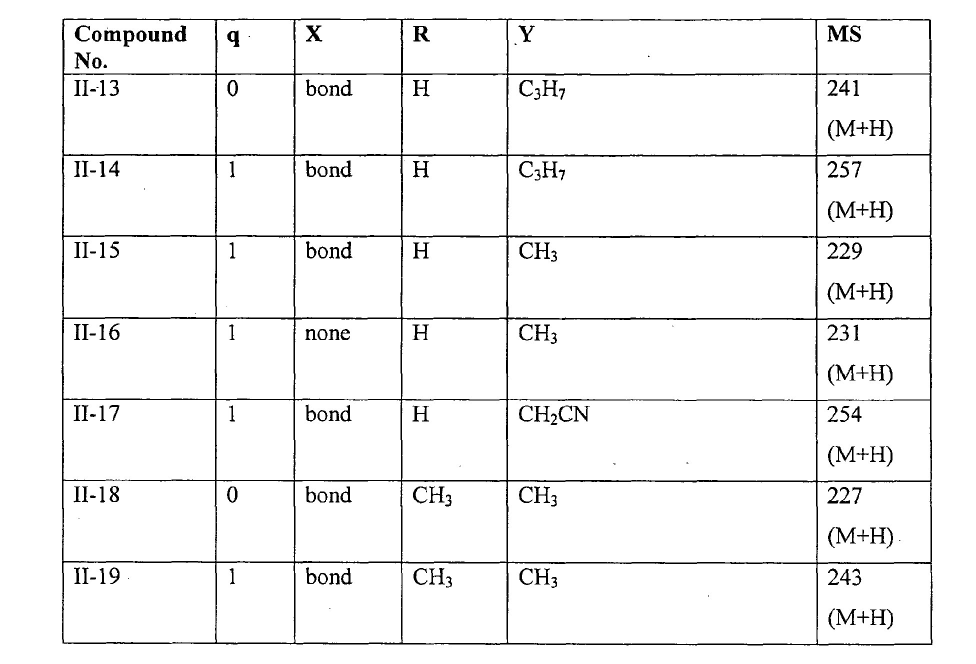

Compounds of the present invention can be generated following various synthetic protocols as shown below.

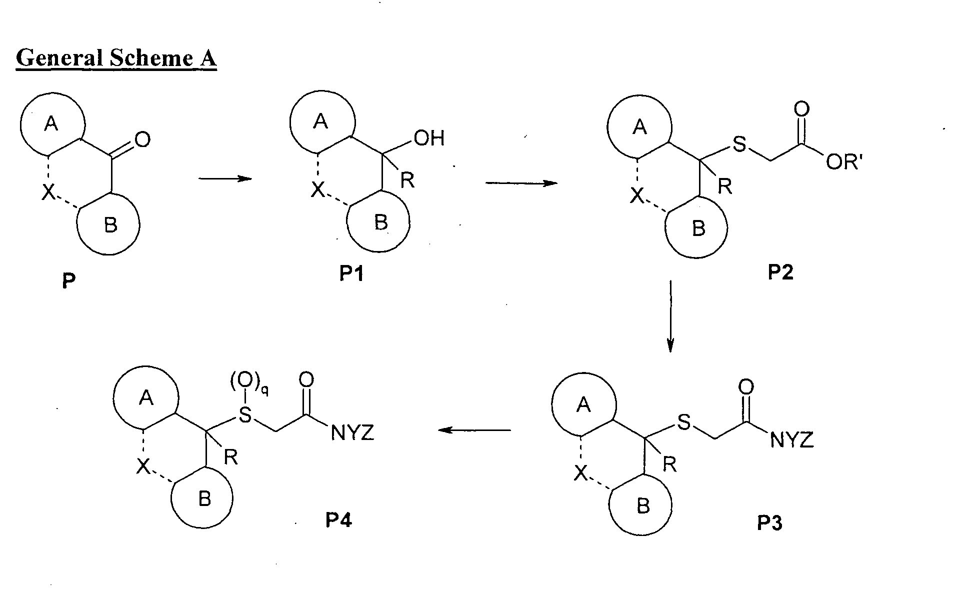

### General synthetic procedure for compounds P2, P3, and P4:

Synthesis of compound **P4** could be initiated from compound **P.** Thus, compound **P** is converted to corresponding hydroxyl compound **P1** by reaction with an alkyl magnesium halide (RMgX) reagent. Compound **P1** could then be reacted with a thiol compound (containing a terminal carbalkoxy group), in presence of an organic acid, e.g. trifluoroacetic acid, to generate compound **P2**. Conversion of compound **P2** to compound **P3** via the intermediacy of a carboxylic acid moiety could be affected by basic hydrolysis with LiOH followed by amidation reaction. Oxidation of compound **P3** by an appropriate agent e.g. hydrogen peroxide in acidic medium or m-chloroperbenzoic acid in an organic solvent produces compound **P4**.

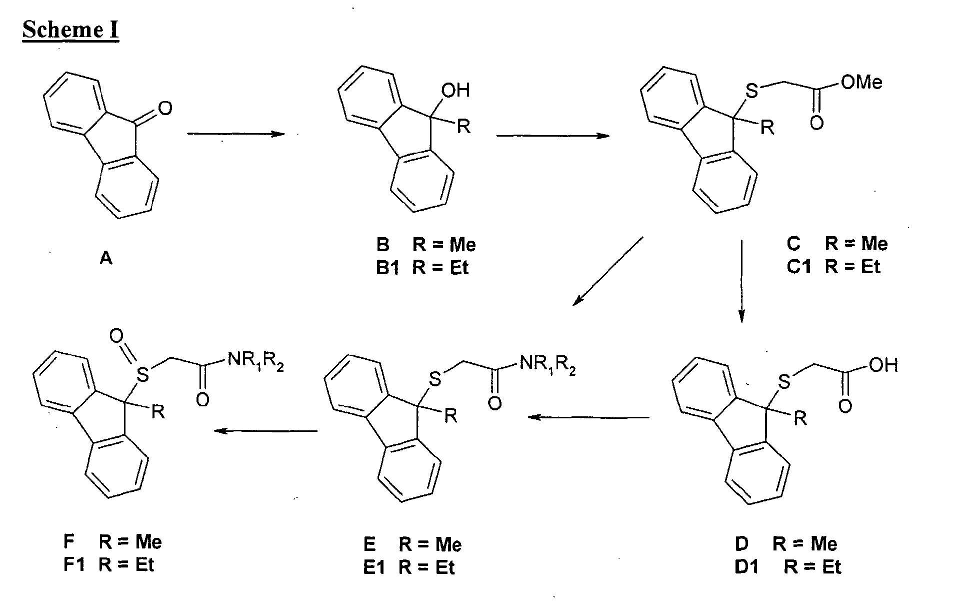

### Preparation of compound B

To a stirring solution of compound **A** (5 g, 27.7 mmol) in dry THF (60 mL) at 0 °C, under N₂, was added MeMgBr (3M in diethyl ether, 9.24 mL). The cooling bath was removed and the mixture was stirred for an additional 1.5 h. More MeMgBr (0.8 ml) was added to the reaction mixture followed by additional stirring for another 3 h. The reaction was carefully quenched with ice-water and extracted into ethyl acetate (3 x 100 mL). The combined organic layers were washed with brine (1 x 50 mL), dried (MgSO₄), and concentrated to yield compound **B** (4.76g): ¹H-NMR (DMSO-d₆) δ 7.73 (d, 2H), 7.53 (dd, 2H), 7.36 - 7.28 (m, 4H), 5.51 (s, 1H), 1.57 (s, 3H).

### Preparation of compound B 1

This compound was prepared following the similar procedure as described previously for the synthesis of compound **B,** except that EtMgBr was used in place of MeMgBr. Thus, starting with 5 g of compound **A,** 2.69 g of compound **B1** was obtained: ¹H-NMR (DMSO-d₆) δ 7.73 (d, 2H), 7.47 (d, 2H), 7.36 - 7.27 (m, 4H), 5.51 (s, 3H), 2.02 (q, 2H), 0.42 (t, 3H).

### Preparation of compound C

A mixture of compound **B** (1.5g, 7.6 mmol), methyl thioglycolate (0.68 mL, 7.6 mmol) and trifluoroacetic acid (0.58 mL, 7.6 mmol) in CH₂Cl₂ (15 mL) was stirred at room temperature for 18 h, quenched with sat. sodium bicarbonate and extracted into CH₂Cl₂ (3 x 50 mL). The combined organic layers were washed with brine (1 x 50 mL), dried (MgSO₄) and concentrated to give a crude product that was purified by silica gel column chromatography (hexane:ethyl acetate :: 8:1) to yield 1.8 g of product as a pale yellow solid: ¹H-NMR (DMSO-d₆) δ 7.84 - 7.82 (m, 2H), 7.57-7.55 (m, 2H), 7.41 - 7.34 (m, 4H), 3.21 (s, 3H), 2.58 (s, 2H), 1.71 (s, 3H).

### Preparation of compound C1

This compound was prepared following the similar procedure as described previously for the synthesis of compound **C**, except that compound **B1** was used in place of compound **B**. Thus, starting with 2.68 g of compound **B1,** 3.7 g of product was obtained: ¹H-NMR (DMSO-d₆) δ 7.84 - 7.82 (m, 2H), 7.51 - 7.49 (m, 2H), 7.41 - 7.34 (m, 4H), 3.21(s, 3H), 2.58 (s, 2H), 2.23 (q, 2H), 0.35 (t, 3H).

### Preparation of compound D

To a solution of compound **C** (0.5 g, 1.75 mmol) in methanol (6 mL) at room temperature was added LiOH•H₂O (0.088 g, 2.1 mmol) in water (2 mL). The reaction mixture was stirred at this temperature for 3 h and then at 60 °C for 1 h. It was then concentrated, diluted with water (20 mL), washed with diethyl ether (2 x 15 mL), acidified (pH ~2) with 2N HCl and extracted with ethyl acetate (2 x 50 mL). The combined organic layers were washed with brine (1 x 20 ml), dried (MgSO₄) and concentrated *in vacuo* to give 0.41 g of product: ¹H-NMR (CDCl₃): δ 9.30 (bs, 1H), 7.70 (d, 2H), 7.60 (d, 2H), 7.30 (m, 4H), 2.50 (s, 2H), 1.80 (s, 3H).

### Example I-1

### Synthesis of compound I-1 (compound E, wherein NR₁R₂ = NH₂)

To a refluxing solution of compound **D** (2.35 g, 8.6 mmol) in benzene (18 mL) was added thionyl chloride (2.6 mL, 34.7 mmol) dropwise. The reaction mixture was heated for 1 h, concentrated *in vacuo*, dissolved in dichloromethane (50 mL) and treated with 28% NH₄OH (10 mL) at room temperature. The mixture was vigorously stirred for 1h and the layers were separated. The aqueous layer was extracted with dichloromethane (1 x 50 mL). The combined organic layers were washed with water (2 x 20 mL), brine (1 x 20 ml), dried (MgSO₄), and concentrated to give a residue that on trituration with ether generated 1.54 g of product: ¹H-NMR (CDCl₃): δ 7.70 (d, 2H), 7.60 (d, 2H), 7.30 (m, 4H), 5.80 (bs, 1H), 5.10 (bs, 1H), 2.50 (s, 2H), 1.80 (s, 3H).

### Example I-2

### Synthesis of compound I-2 (compound E, wherein NR₁R₂ = NMe₂)

This compound was prepared following the similar procedure as described in Example **I-1** wherein dimethylamine gas was used in place of 28% NH₄OH in the amidation step and the final product was purified by silica gel column chromatography (ethyl acetate:hexanes :: 1:1). Thus, starting from 2.3 g of compound **D,** 1.8 g of product was obtained: ¹H-NMR (CDCl₃): δ 7.70-7.60 (m, 4H), 7.30 (m, 4H), 2.70 (s, 3H), 2.60 (s, 3H), 2.50 (s, 2H), 1.80 (s, 3H).

### Example I-3

### Synthesis of compound I-3 (compound E, wherein NR₁R₂ = NH-(s)-CH(Me)CONH₂)

To a solution of compound **D** (2 g, 7.35 mmol) in DMF (10 mL) at room temperature was successively added 2-(1H-benzotriazol-1-yl)-1,1,3,3-tetramethyluronium tetrafluoroborate ("TBTU")(1.2 eqv) and *N*-methylmorpholine ("NMM")(1 mL). The mixture was stirred for 10 min, treated dropwise with a mixture of alanine hydrochloride (1.37 g, 11 mmol) and NMM (2 mL) in DMF (15 ml) and stirred overnight. The reaction mixture was then diluted with water and extracted with ethyl acetate (3 x 50 mL). The combined organic layers were washed with water (3 x 20 mL), brine (1 x 20 mL), dried (MgSO₄) and concentrated to give a crude solid that, on trituration with ether, generated 2.50 g of product: ¹H-NMR (DMSO-d₆): δ 7.60 - 7.10 (m, 8H), 7.00 (s, 1H), 6.80 (s, 1H), 3.70 (m, 1H), 2.30 (m, 2H), 2.20 (s, 1H), 1.50 (s, 3H), 0.80 (d, 3H).

### Example I-4

### Synthesis of compound I-4 (compound E, wherein NR₁R₂ = N-pyrrolidinyl)

A mixture of compound **C** (1.76 g, 6.2 mmol), pyrrolidine (2.58 mL, 31 mmol) and methanol (10 mL) was stirred at room temperature for 65 h and concentrated to generate a crude product that was purified by silica gel column chromatography (hexane: ethyl acetate :: 1:1) to yield 1.32 g of product: ¹H-NMR (DMSO-d₆) δ 7.85 - 7.83 (m, 2H), 7.62 - 7.60 (m, 2H), 7.42 - 7.35 (m, 4H), 3.00 (t, 2H), 2.83 (t, 2H), 2.54 (s, 2H), 1.72 (s, 3H), 1.69 - 1.59 (m, 4H).

### Example I-5

### Synthesis of compound I-5 (compound E1, wherein NR₁R₂ = NH₂)

A mixture of compound **C1** (1.24 g, 4.2 mmol), methanol (5 mL) and NH₃ gas was maintained at 50 °C in a sealed tube for 20 h, cooled to room temperature, recharged with NH₃ gas and kept at 50 °C for an additional 20 h. The reaction mixture was then concentrated and triturated with ether to yield 0.94 g of product. This material was used in the next step without any further purification: ¹H-NMR (DMSO-d₆) δ 7.86 - 7.82 (m, 2H), 7.65 - 7.52 (2H), 7.42 - 7.35 (m, 4H), 7.12 (br s, 1H), 6.83 (br s, 1H), 2.48 (s, 2H), 2.25 (q, 2H), 0.35 (t, 3H).

### Example I-6

### Synthesis of compound I-6 (compound E1, wherein NR₁R₂ = N-pyrrolidinyl)

This compound was prepared following the similar procedure as described previously in Example **I-5** wherein pyrrolidine was used in place of 28% NH₄OH in the amidation step that was carried out at room temperature and the final product was purified by silica gel column chromatography (ethyl acetate:hexanes :: 1:1). Thus, starting from 1.29 g of compound **I-2,** 1.38 g ofproduct was obtained: ¹H-NMR (DMSO-d₆) δ 7.86-7.83 (m, 2H), 7.56 - 7.54 (m, 2H), 7.41 - 7.35 (m, 4H), 3.01 (t, 2H), 2.84 (t, 2H), 2.24 (q, 2H), 2.50 (m, 2H), 1.68 - 1.59 (m, 4H), 0.37 (t, 3H).

### Example I-7

### Synthesis of representative compound I-7 (compound F, wherein NR₁R₂ = NH₂)

A mixture of compound **I-1** (1.18 g, 4.3 mmol) in gl. acetic acid (10 mL) and 50% aqueous H₂O₂ (1.1 eqv) was stirred at room temperature 2 h, treated with additional peroxide (0.2 eqv) and stirred for another 1 h. It was then diluted with water (20 mL) and extracted with ethyl acetate (3 x 75 mL). The combined organic layers were washed with 2% aq. sodium bicarbonate (2 x 10 mL), water (1 x 10 mL), and brine (1 x 10 mL), dried (MgSO₄), and concentrated to give a crude solid that on trituration with ether generated 1.09 g of product: ¹H-NMR (DMSO-d₆): δ 7.80 (t, 2H), 7.50-7.30 (m, 7H), 6.90 (b, 1H), 2.10 - 1.90 (q, 2H), 1.80 (s, 3H).

### Example I-8

### Synthesis of compound I-8 (compound F1, wherein NR₁R₂ = NH₂)

Compound **I-5** was oxidized to give the product following the same procedure as described in Example I-7; ¹H-NMR (DMSO-d₆): δ 8.00 (m, 2H), 7.50 (m, 6H), 7.40 (d, 1H), 7.10 (d, 1H), 2.50 (m, 2H), 2.20 (dd, 2H), 0.50 (t, 3H).

### Example I-9

### Synthesis of compound I-9 (compound F, wherein NR₁R₂ = NMe₂)

Compound **I-2** was oxidized to give the product following the same procedure as described in Example I-7; ¹H-NMR (DMSO-d₆): δ 8.20-7.50 (series of m, 8H), 2.80 (s, 3H), 2.50 (s, 3H), 2.60 - 2.30 (2 d, 2H), 2.10 (s, 3H).

### Example I-10

### Synthesis of compound I-10 (compound F, wherein NR₁R₂ = NH-(s)-CH(Me)CONH₂)

Compound **I-3** was oxidized to give the product (mixture of diastereomers) following the same procedure as described in Example I-7; ¹H-NMR (DMSO-d₆): δ 8.40-7.50 (m, 8H), 7.40 - 6.80 (2 sets of d, 2H), 4.00 (m, 1H), 3.20 (q, 1H), 2.50-2.30 (m, 2H), 1.80 (s, 3H), 1.10 (m, 3H).

### Example I-11

### Synthesis of compound I-11 (compound F, wherein NR₁R₂ = N-pyrrolidinyl)

Compound **I-4** was oxidized to give the product following the same procedure as described in Example I-7; ¹H-NMR (DMSO-d₆): δ 8.00 (m, 2H), 7.50 (m, 6H), 3.10 - 2.70 (series of m, 4H), 2.20 (dd, 2H), 1.90 (s, 3H), 1.70 (m, 4H).

### Example I-12

### Synthesis of compound I-12 (compound F1, wherein NR₁R₂ = N-pyrrolidinyl)

Compound **I-6** was oxidized to give the product following the same procedure as described in Example I-7; ¹H-NMR (DMSO-d₆): δ 8.00 (m, 2H), 7.50 (m, 6H), 3.10 (m, 2H), 2.70 (m, 4H), 2.20 (dd, 2H), 1.70 (m, 4H), 0.50 (t, 3H).

### Example I-13

### Synthesis of compound I-13

Starting with benzophenone in place of compound **A** in **Scheme I,** the product was prepared following the same multi-step synthetic sequence as described in the previous examples; ¹H-NMR (DMSO-d₆): δ 7.50 (b, 1H), 7.40-7.20 (m, 10H), 7.10 (b, 1H), 2.80 (dd, 2H), 1.80 (s, 3H).

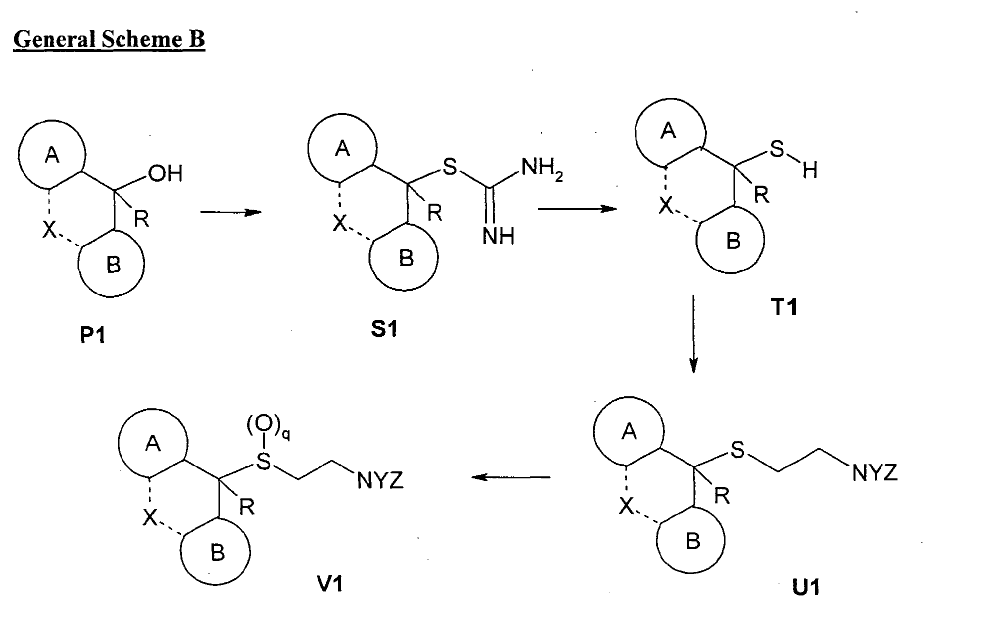

### General synthetic procedure for compounds in General Scheme B:

Synthesis of various compounds can be initiated from compound **P1**. Thus, compound **P1** is converted to corresponding compound **S1** by reaction with thiourea in an acidic medium e.g. HBr. Compound **S1** could then be hydrolyzed to corresponding thiol compound **T1** in a basic hydrolysis step. In situ alkylation of compound **T1** by an appropriate alkylating agent containing a terminal amino group generates compound **U1** that could further be derivatized in the amino position in subsequent steps. Oxidation of compound **U1** by an appropriate agent e.g. hydrogen peroxide in acidic medium or m-chloroperbenzoic acid produces compound **V1**.

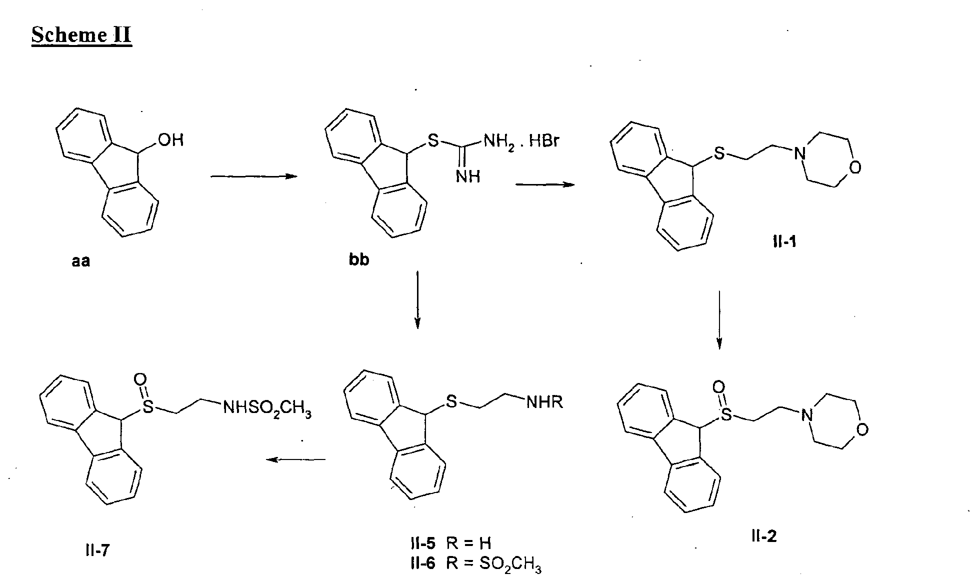

### Preparation of compound bb

Synthesis of compound **bb** had been disclosed in U.S. Pat. No. 6,492,396 which is incorporated herein by reference in its entirety.

### Example II-1

### Synthesis of compound II-1

To a mixture of compound **bb** (2.13 g, 6.89 mmol) in water (5 mL) at 70 °C was added a mixture of 4-(2-chloroethyl)morpholine hydrochloride (1.53 g, 8.2 mmol) in water (5 mL) and 10N NaOH (3 mL). The reaction mixture was heated at 110 °C for 1 h, cooled and extracted into ether (3 x 50 mL). The combined organic layers were washed with water (1 x 15 mL), brine (1 x 15 ml), dried (MgSO₄) and concentrated to generate a crude product that was purified by flash chromatography (silica gel; solvent: ethyl acetate: hexane :: 2 : 3) to yield 1.48 g of compound **II-1:** ¹H-NMR (CDCl₃): δ 7.70 (m, 4H), 7.30 (m, 4H), 4.90 (s, 1H), 3.50 (m, 4H), 2.20 - 1.90 (m, 8H).

### Example II-2

### Synthesis of compound II-2

To a cooled (-15 °C) solution of compound **II-1** (1.45 g, 4.66 mmol) in dichloromethane (15 mL) was added *m*-chloroperbenzoic acid (77%, 0.8 g, 4.66 mmol) in portions. The reaction mixture was stirred for 1 h, quenched with 2% aq. sodium bicarbonate (50 ml), and diluted with dichloromethane (100 ml). The separated organic layers were washed with 2% aq. sodium bicarbonate (2 x 20 ml), water (1 x 20 mL), and brine (1 x 20 ml), dried (MgSO₄), filtered and concentrated to give a crude product. It was purified by flash chromatography (silica gel; ethyl acetate followed by methanol:dichloromethane:: 5:95) to yield an oil that on trituration with ether generated 0.067 g of compound **II-2:** ¹H-NMR (CDCl₃): δ 7.90 - 7.70 (m, 3H), 7.60 - 7.30 (m, 5H), 5.40 (s, 1H), 3.60 (m, 3H), 2.50 (m, 1H), 2.30 - 2.20 (m, 5H), 1.80 - 1.60 (m, 3H).

### Example II-3

### Synthesis of compound II-3

This compound was prepared following the same procedure as described in Examples II-1 and II-2, except that terminal morpholinyl group was replaced by a pyrrolidinyl group; ¹H-NMR (CDCl₃): δ 8.00 - 7.30 (series of m, 8H), 5.60 (s, 1H), 3.20 - 2.40 (series of broad m, 8H), 1.70 (broad, 4H).

### Example II-4

### Synthesis of compound II-4

This compound was prepared following the same procedure as described in Examples II-1 and II-2, except that terminal morpholinyl group was replaced by a piperidinyl group; ¹H-NMR (CDCl₃): δ 8.10 - 7.30 (series ofm, 8H), 5.60 (s, 1H), 3.20 - 2.40 (series of broad m, 8H), 1.70 (broad, 4H), 1.60 (broad, 2H).

### Example II-5

### Synthesis of compound II-5

This compound was prepared following the same procedure as described in Example II-1 wherein 2-chloro-ethylamine hydrochloride was used in place of 4-(2-chloroethyl)morpholine hydrochloride as one of the reactants. This material was directly used in the next step.

### Example II-6

### Synthesis of compound II-6

To a mixture of compound **II-5** (0.73 g, 3.04 mmol) and triethylamine (0.47 ml, 3.4 mmol) in dichloromethane (10 mL) at 0 °C was added methanesulfonyl chloride (0.26 mL, 3.35 mmol). The cooling bath was removed and the reaction mixture was stirred at room temperature for 1 h, treated with 2N HCl (20 mL) and extracted into dichloromethane (2 x 25 mL). The combined organic layers were washed with water (1 x 10 mL), brine (1 x 10 mL), dried (MgSO₄) and concentrated to give 0.97 g of compound **II-6** that was immediately taken into next step.

### Example II-7

### Synthesis of compound II-7

To a mixture of compound **II-6** (0.97 g, 3.03 mmol) in glacial acetic acid (10 mL) at room temperature was added H₂O₂ (50% in water, 0.247 mL). The mixture was stirred for 0.5 h, diluted with ice-water (100 mL) and stirred for an additional 0.5 h. The separated solid was filtered and washed several times with water and ether successively, and dried under high vacuum to generate 0.56 g of compound **II-7:** ¹H-NMR (DMSO-d₆): δ 7.80 (t, 2H), 7.60 (d, 1H), 7.50 (d, 1H), 7.30 (m, 2H), 7.20 (m, 3H), 5.50 (s, 1H), 2.90 (m, 2H), 2.60 (s, 3H), 1.90 (m, 2H).

### Example II-8

### Synthesis of compound II-8

This compound was prepared following the same synthetic scheme as described in Examples II-6 and II-7 except that an acetamido group was employed at the terminus; ¹H-NMR (DMSO-d₆): δ 8.10 - 7.30 (series ofm, 8H), 5.60 (s, 1H), 3.30 (s, 1H), 3.20 (m, 2H), 2.10 (m, 2H), 1.80 (s, 3H).

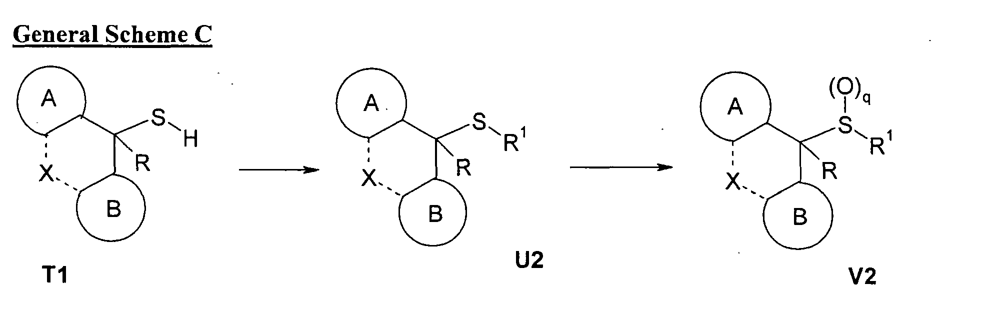

### General synthetic procedure for compounds in General Scheme C

Synthesis of compound **V2** could be initiated from compound **T1**. Thus, alkylation of compound **T1** by an appropriate alkylating agent in presence of a base generates compound **U2** that on oxidation by an appropriate agent e.g. hydrogen peroxide in acidic medium or m-chloroperbenzoic acid produces compound **V2**.

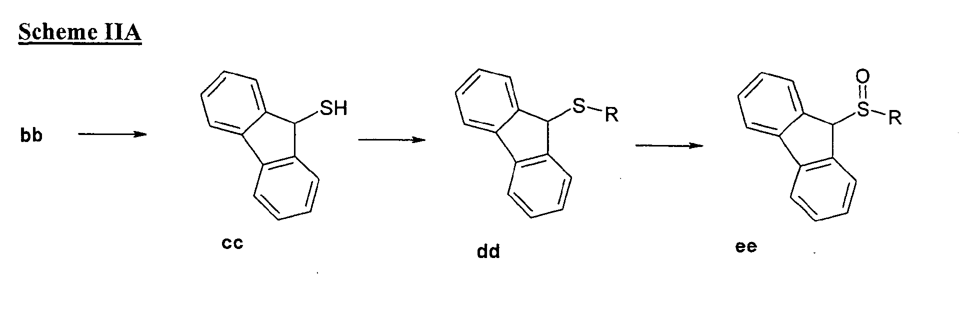

### Example II-9

### Synthesis of compound II-9 (compound ee wherein R = -CH₂CH=CH₂)

A mixture of compound **bb** (14.12 g, 44 mmol), 10 N NaOH (14.9 mL) and water (109 mL) was heated at 70 °C for 0.5 h, cooled, diluted with ice-water, acidified (pH ~ 2) and extracted into ethyl acetate (3 x 100 mL). The combined organic layers were washed with water (1 x 50 mL) and brine (1 x 50 mL), dried (MgSO₄) and concentrated to yield 9.45 g of compound cc that was directly taken into next step without any further purification; ¹H-NMR (DMSO-d₆) δ 7.89 (d, 2H), 7.76 (d, 2H), 7.43 (m, 4H), 5.21 (d, 1H), 3.55 (d, 1H).

Thus, a mixture of compound **cc** (2 g, 10.1 mmol) in methanol (16 mL) and sodium methoxide (0.5 M in methanol, 20.2 mL) was heated at 60 °C for 0.5 h, treated with allyl iodide (4.66 mL, 50.5 mmol), continued heating for an additional 0.5 h, cooled, and quenched with ice-water. It was then acidified (pH ~ 2) and extracted into ethyl acetate (3 x 50 mL). The combined organic layers were washed with water (1 x 50 mL) and brine (1 x 50 mL), dried (MgSO₄) and concentrated to yield a crude material. This material was stirred in pet. ether (20 mL) and filtered. The filtrate, upon concentration, provided 1.97 g of compound **dd** (R = -CH₂CH=CH₂) which was oxidized by 50% H₂O₂ to give compound **II-9** following the previously described procedure in Example I-10; ¹H-NMR(DMSO-d₆) δ 8.00 - 7.20 (series ofm, 8H), 5.60 (s, 1H), 5.50 (m, 1H), 5.50 (m, 2H), 2.90 (m, 2H).

### Example II-10

### Synthesis of compound II-10 (compound ee wherein R = -CH₂C(Me)=CH₂)

This compound was prepared following the same scheme as in Example II-9, except that a 2-methyl propylene group was employed at the terminus; ¹H-NMR (DMSO-d₆) δ 8.00 - 7.20 (series of m, 8H), 5.60 (s, 1H), 4.90 (s, 1H), 4.60 (s, 1H), 2.60 (dd, 2H), 1.50 (s, 3H).

### Example II-11

### Synthesis of compound II-11 (compound dd wherein R = -CH₂CHMe₂)

A mixture of compound **cc** (2 g, 10.1 mmol) in methanol (16 mL) and sodium methoxide (0.5 M in methanol, 20.2 mL) was heated at 60 °C for 0.5 h, treated with 1-iodo-2-methylpropane (6 mL, 50.5 mmol), heated for an additional 0.5 h, cooled, and quenched with ice-water. It was then acidified (pH ~ 2) and extracted into ethyl acetate (3 x 50 mL). The combined organic layers were washed with water (1 x 50 mL) and brine (1 x 50 mL), dried (MgSO₄) and concentrated to yield a crude material. This material was stirred in pet. ether (20 mL) and filtered. The filtrate, upon concentration, provided 2.21 g of compound **II-11** that was directly used in the next step: ¹H-NMR (DMSO-d₆) δ 7.86 (d, 2H), 7.64 (d, 2H), 7.42 (m, 4H), 5.13 (s, 1H), 1.90 (d, 2H), 1.36 (m, 1H), 0.74 (s, 3H), 0.72 (s, 3H).

### Example II-12

### Synthesis of compound II-12 (compound ee wherein R = -CH₂CHMe₂)

To a cooled (ice-bath) solution of compound **II-11** (1 g, 3.9 mmol) in gl. acetic acid (4 mL) was added 50% H₂O₂ (0.27 mL). The reaction mixture was stirred for 1 h, diluted with ethyl acetate and concentrated to give a crude product that was purified by flash chromatography (silica, solvent-gradient: hexane:ethyl acetate :: 4:1 to ethyl acetate) to generate 0.71 g of compound **II-12:** ¹H-NMR (DMSO-d₆) δ 7.97 (t, 2H), 7.73 (d, 1H), 7.63 (d, 1H), 7.52 (m, 2H), 7.38 (m, 2H), 5.60 (s, 1H), 1.89 (dd, 1H), 1.76 (m, 1H), 1.66(dd, 1H), 0.78 (d, 3H), 0.76 (d, 3H).

### Example II-13

### Synthesis of compound II-13 (compound dd wherein R = C₃H₇)

This compound was prepared following the same procedure as described before for the synthesis of compound II-11, except that n-propyl iodide was utilized as an alkylating agent. It was immediately used in the synthesis of compound II-14.

### Example II-14

### Synthesis of compound II-14 (compound ee wherein R = C₃H₇)

Utilizing compound II-I3, this compound was prepared following the same procedure as described before for the synthesis of compound II-12; ¹H-NMR (DMSO-d₆) δ 8.00 - 7.20 (series of m, 8H), 5.60 (s, 1H), 1.90 (2 sets of m, 2H), 1.50 (m, 2H), 0.80 (t, 3H).

### Example II-15

### Synthesis of compound II-15 (compound ee wherein R = CH₃)

This compound had been described by: Kice, J. L., Lotey, H. J *Org. Chem*. **1988,** *53*, 3593; this reference has been included herein in its entirety.

### Example II-16

### Synthesis of compound II-16

This compound had been described by Mizuno, H., Matsuda, M., Ino, M. *J. Org. Chem*. **1981,** *46*, 520; this reference has been included herein in its entirety.

### Example II-17

### Synthesis of compound II-14 (compound ee wherein R = CH₂CN)

This compound had been described by: Kice, J. L., Lotey, H. *J. Org. Chem*. **1988,** *53*, 3593; this reference has been included herein in its entirety.

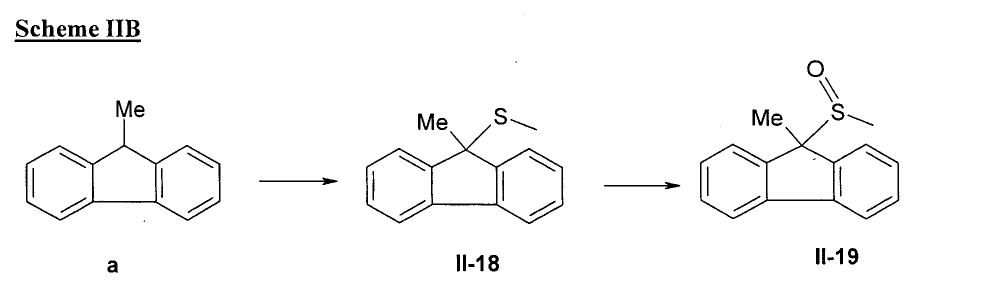

### Example II-18

### Synthesis of compound II-18

To a cooled (-78 °C) solution of compound **a** (3.43g, 19 mmol) in anhydrous THF (60 mL) was added n-butyl lithium in hexanes (2.5 M, 9.1 mL, 23 mmol). The reaction mixture was stirred for an additional 0.5 h, treated with dimethyl disulfide (2.54 mL, 29 mmol) in two portions over a period of 0.5 h, and stirred for another 0.5 h. It was then quenched with ice-water (50 mL) and extracted into ethyl acetate (2 x 50 mL). The combined organic layers were washed with brine (1 x 50 mL), dried (MgSO₄) and concentrated to give a crude material that was purified by silica-gel column chromatography (solvent: hexanes) to yield 3.33 g of compound **II-18** (yellow solid): ¹H-NMR (DMSO-d₆) δ 7.85 - 7.82 (m, 2H), 7.58 - 7.55 (m, 2H), 7.40 - 7.35 (m, 4H), 1.72 (s, 3H), 1.32 (s, 3H). The method was an adaptation from a procedure previously described in *J Med Chem* **1986,** *29*,1577, which is incorporated herein by reference in its entirety.

### Example II-19

### Synthesis of compound II-19

To a cooled (-78 °C) solution of compound **II-18** (3.32 g, 14.7 mmol) in CH₂Cl₂ (50 mL) was slowly added a solution of *m*-chloroperbenzoic acid (70-75%, 3.96 g) in CH₂Cl₂ (30 mL). The reaction mixture was stirred for 2 h, treated with an additional 0.8 g of m-chloroperbenzoic acid, and stirred for another 2 h. It was then quenched with sat. NaHCO₃ (50 mL). The organic layer was separated and washed with sat. NaHCO₃ (2 x 50 mL), and water (1 x 50 mL), dried (MgSO₄) and concentrated to give a crude product that was purified by silica gel column chromatography (solvent gradient: 4:1 hexane / ethyl acetate to 2:1 hexane / ethyl acetate) to yield 3.06 g of compound **II-19:** ¹H-NMR (DMSO-d6) δ 8.00 - 7.95 (m, 2H), 7.60 - 7.38 (m, 6H), 1.91 (s, 3H), 1.40 (s, 3H).
Additional compounds encompassed by the present invention include those set forth in the following table. This list is meant to be representative only and is not intended to limit the scope of the invention in any way. The reagents and starting materials are commercially available, or readily synthesized by well-known techniques by one of ordinary skill in the arts. All substituents in the synthetic Schemes, unless otherwise indicated, are as previously defined.

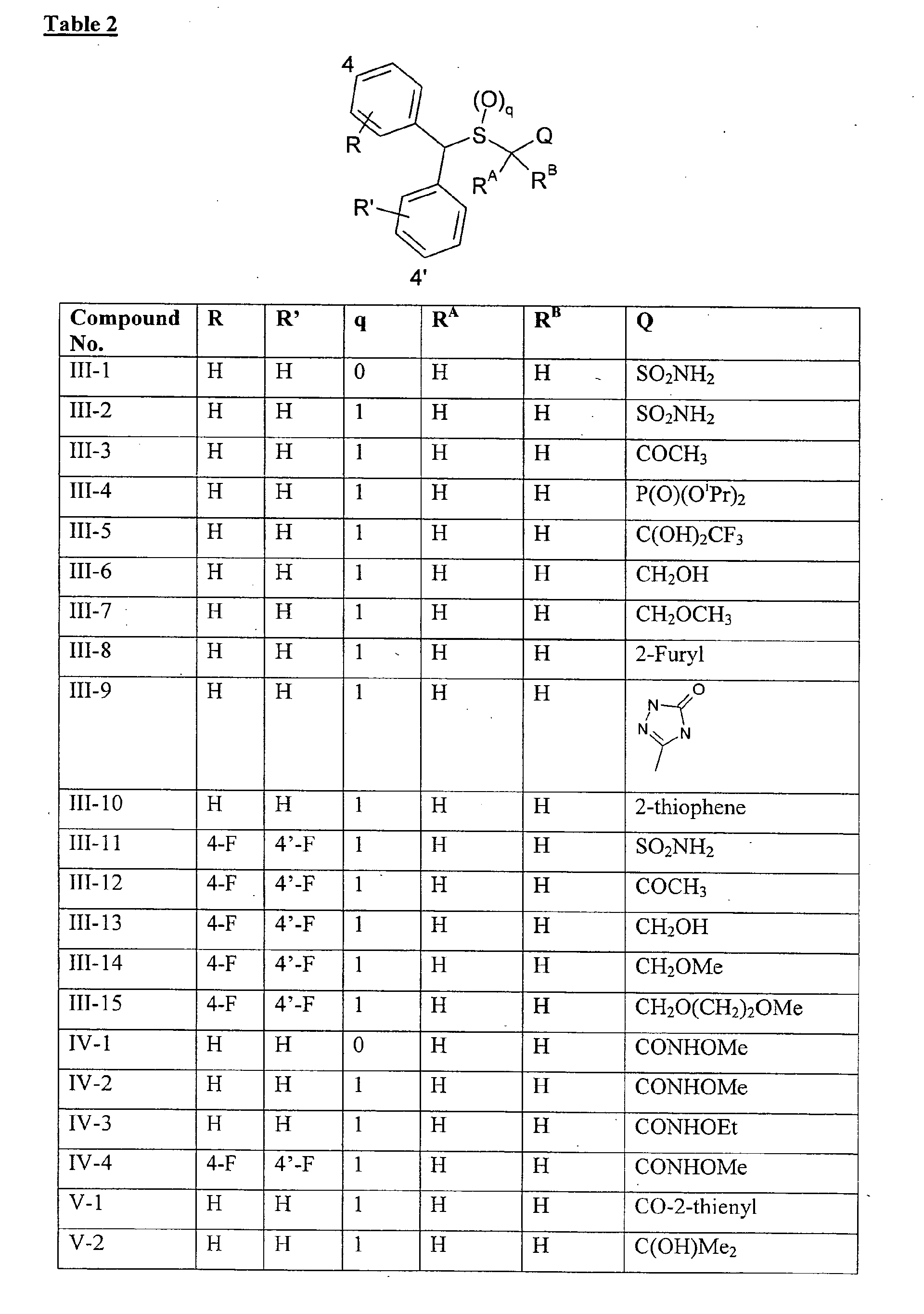

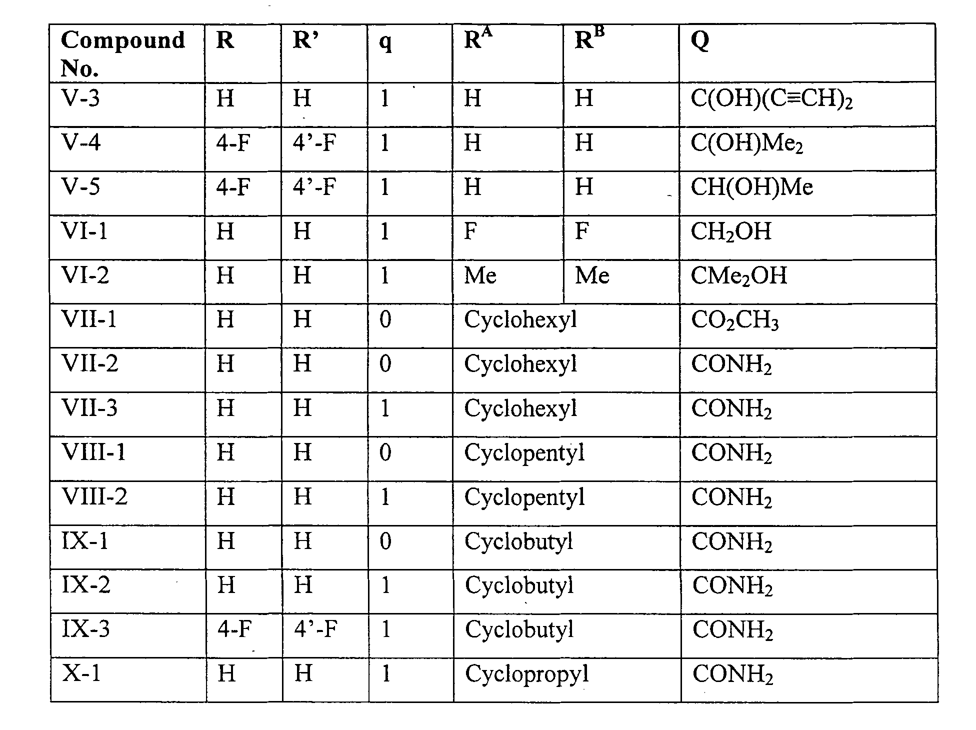

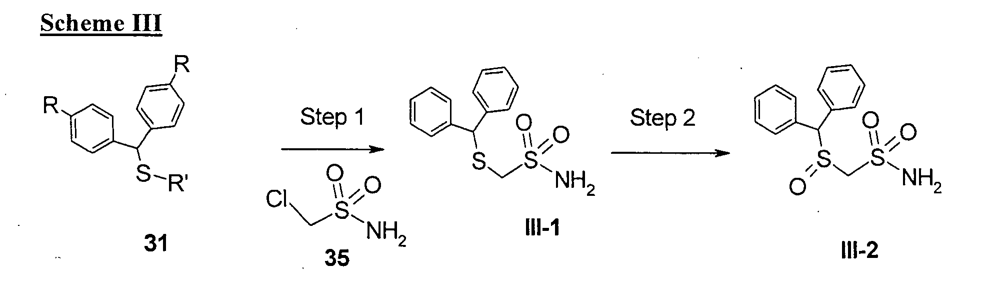

**a** R = H, R' = C(=NH)NH₂.HBr
**b** R = F, R' = C(=NH)NH₂.HBr
**c** R = H, R' = H

Reagents for Step 1, compounds **31a** and **31b:** (i) 10 N NaOH/EtOH/70 °C; (ii) compound **35,** reflux 2h. Step 2: 50% H₂O₂ in water/HOAc/RT or *m*-chloroperbenzoic acid, dichloromethane, 0 °C.
Reagents for Step 1, compound **31c:** NaH/DMF/compound **35**/room temperature to 70 °C.

### Preparation of compounds 31 and 35

The preparation of compounds **31c** (U.S. Pat. No. 4,066,686) and 35 (El-Hewehi, Z.; Runge, F. *J. Prakt. Chem*. **1962,** *16*, 297) were described in the literature, and both references are incorporated herein in their entireties.

### Example III-1

### Synthesis of Compound III-1

To a stirred mixture of NaH (60% in oil, 745 mg, 18.62 mmol) in dry DMF (15 mL) at room temperature and under argon was added dropwise a solution of compound **31c** (3.33g, 16.64 mmol) in dry DMF (3 mL). The mixture was stirred at room temperature for 15 min. The reaction mixture was then treated with compound **35** (2.2 g, 16.98 mmol) followed by heating at 70 °C for 4 h. It was then cooled to room temperature, concentrated at high vacuum, diluted with water and extracted into EtOAc. The combined organic layers were washed successively with water and brine, dried (MgSO₄) and concentrated to give a crude product that was purified by flash chromatography (silica gel, hexane : EtOAc :: 3 :2 ) to yield compound **III-1** (3.9 g): ¹H-NMR (DMSO-d₆): δ 7.18 - 6.8 (series of m, 11H), 6.65 (s, 1H), 5.25 (s, 1H), 2.90 (s, 2H).

### Example III-2

### Synthesis of compound III-2

To a solution of the compound **III-1** (3.88 g, 13.24 mmol) in acetic acid (25 mL) was added hydrogen peroxide (50% solution in water, 910 µL). The reaction mixture was stirred at room temperature overnight. Solvent was removed and the crude product was stirred in EtOAc, filtered and dried to generate compound **III-2** (1.08 g) m.p.: 165-166 °C, ¹H-NMR (DMSO-d₆): δ 7.54 - 7.35 (series ofm, 12H), 5.52 (s, 1H), 4.23 (d, 1H), 3.93 (d, 1H). MS: 331.91 (M + Na),

### Example III-3

### Synthesis of compound III-3

Utilizing compound **31c** and chloroacetone in first step, compound **III-3** was synthesized; mp.: 81 - 82 °C; ¹H-NMR (DMSO-d₆): δ 7.56-7.32 (series of m, 10H), 5.34 (s, 1H), 3.66 (dd, 2H), 2.13 (s, 3H). MS: 294.99 (M+Na)

### Example III-4

### Synthesis of Compound III-4

Utilizing compound **31c** and diisopropylbromomethyl phosphonate in first step, compound **III-4** was synthesized; mp: 127-128 °C, ¹H-NMR (DMSO-d₆): δ 7.53-7.34 (series of m, 10H), 5.42 (s, 1H), 4.58 (m, 2H), 2.94 (m, 2H). MS: 394.79 (M + H).

### Example III-5

### Synthesis of Compound III-5

Utilizing compound **31c** and 3-bromo-1,1,1-trifluoro-propan-2-one in first step, compound **III-5** was synthesized; m.p.: 121-122 °C; ¹H-NMR (DMSO-d₆): δ 7.72-7.32 (series of m, 12H), 5.37 (s, 1H), 3.02 (d, 1H), 2.69 (d, 1H). MS: 366.92 (M + Na).

### Example III-6

### Synthesis of compound III-6

Utilizing compound **31c** and 2-chloroethoxytrimethylsilane in first step, compound **III-6** was synthesized; mp.: 134 °C, ¹H-NMR (DMSO-d₆): δ 7.64-7.30 (series ofm, 10H), 5.24 (s, 1H), 4.94 (m, 1H), 3.69 (m, 2H), 2.64 - 2.49 (two sets of m, 2H). MS: 260.98 (M + H).

### Example III-7

### Synthesis of compound III-7

Utilizing compound **31c** and bromoethylmethyl ether in first step, compound **III-7** was synthesized; m.p.: 71-72 °C; ¹H-NMR (DMSO-d₆): δ 7.52-7.31 (series ofm, 10H), 5.21 (s, 1H), 3.51 (m, 2H), 3.31 (s, 3H), 2.75 - 2.53 (two m, 2H). MS: 274.95 (M + H),

### Example III-8

### Synthesis of compound III-8

Utilizing furan-2-yl-methanethiol in place of **31a/b/c,** and bromodiphenylmethane in step 1, compound **III-8** was synthesized; mp.: 102-103 °C, ¹H-NMR (DMSO-d₆): δ 7.71 - 7.33 (series ofm, 11H), 6.45 (s, 1H), 6.44 (d, 1H), 5.29 (s, 1H), 3.97 (d, 1H), 3.71 (d, 1H). MS: 296.89 (M+H).

### Example III-9

### Synthesis of compound III-9

Utilizing compound **31c** and 3-chloromethyl-1,2,4-triazolin-5-one in step 1, compound **III-9** was synthesized; mp.: >300 °C; ¹H-NMR (DMSO-d₆): δ 11.48(s, 2H), 7.65-7.34 (series of m, 10H), 5.42 (s, 1H) 3.75 (d, J = 13.88 Hz, 1H), 3.46 (d, J = 13.91 Hz, 1H). MS: 313.93 (M + H). 3-Chloromethyl-1,2,4-triazolin-5-one was described by Cowden, C. J.; Wilson R. D.; Bishop, B. C., Cottrell, I. F.; Davies, A. J.; Dolling, U-H. *Tetrahedron Letters,* **2000**, *41*, 8661. This reference has been incorporated herein in its entirety.

### Example III-10

### Synthesis of compound III-10

Utilizing thien-2-yl-methanethiol and bromodiphenylmethane in step 1, compound **III-10** was synthesized; m.p.: 122 - 124 °C; ¹H-NMR (DMSO-d₆): δ 7.67-7.34 (series of m, 11H), 7.03 (m, 1H), 6.95 (m, 1H), 5.26 (ms, 1H), 4.17 (d, 1H), 3.79 (d, 1H). MS: 312.85 (M+H),

### Example III-11

### Synthesis of compound III-11

Utilizing compound **31b** and chloromethanesulfonamide in step 1, compound **III-11** was synthesized; mp: 92-94 °C, ¹H-NMR (DMSO-d₆): δ 7.57-7.26 (two sets ofm, 10H), 5.59 (s, 1H), 4.27 (d, 1H), 3.90 (d, 1H). MS: 367.86 (M+Na),

### Example III-12

### Synthesis of compound III-12

Utilizing compound **31b** and chloroacetone in step 1, compound **III-12** was prepared; mp : 96 - 97 °C;- ¹H-NMR (DMSO-d₆): δ 7.56-7.52 (m, 10H), 7.28-7.05 (m, 4H), 5.40 (s, 1H), 3.76 (d, 1H), 3.61 (d, 1H), 2.15 (s, 3H). MS: 330.95 (M + Na),

### Example III-13

### Synthesis of compound III-13

Utilizing compound **31b** and 2-chloroethoxytrimethylsilane in step 1, compound **III-13** was prepared; mp: 91 - 92 °C; ¹H-NMR (DMSO-d₆): δ 7.56 - 7.51 (m, 4H), 7.27 - 7.21 (m, 4H), 5.33 (s, 1H), 4.97 (t, 1H), 3.70 (m, 2H), 2.67 - 2.60 (m, 1H), 2.50 - 2.43 (m, 1H). MS: 318.96 (M + Na).

### Example III-14

### Synthesis of compound III-14

Utilizing compound **31b** and 2-bromoethylmethyl ether in step 1, compound **III-14** was prepared; mp: 83 - 85 °C, ¹H-NMR (DMSO-d₆): δ 7.56-7.04 (2m, 8H), 5.34 (s, 1H), 3.61 (m, 2H), 3.22 (s, 3H), 2.76 (m, 1H), 2.51 (m, 1H). MS: 310.91 (M + H).

### Example III-15

### Synthesis of compound III-15

Utilizing compound **31b** and 1-bromo-2-(2-methoxyethoxy)ethane in step 1, compound **III-15** was prepared; mp: 41 - 42 °C; ¹H-NMR (DMSO-d₆): δ 7.56-7.22 (two m, 8H), 5.37 (s, 1H), 3.69 (m, 2H), 3.50 (m, 2H), 3.43 (m, 2H), 3.31 (s, 3H), 2.76 (m, 1H), 2.50 (m, 1H). MS: 376.93 (M+Na). 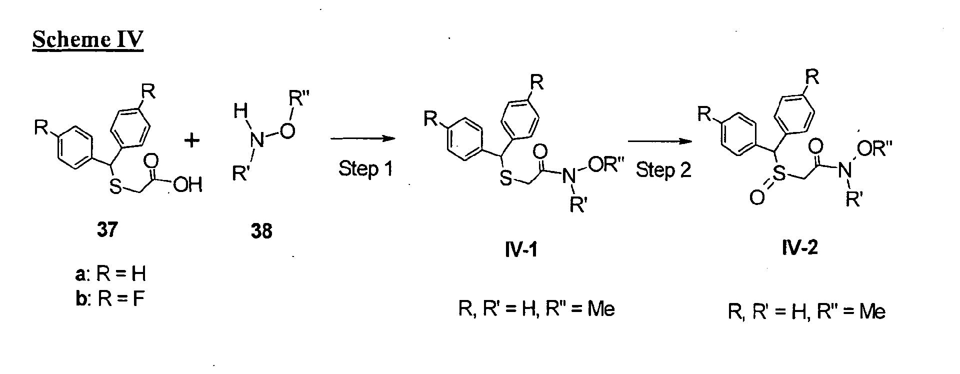
Reagents for Step 1: TBTU/DMF/room temperature or HOBT•NH₃/EDCI
Reagent for Step 2: 50% H₂O₂ in water/HOAc/room temperature.

### Preparation of compound 37a

The preparation of compound **37a** was described in U.S. Pat. No. 4,006,686, which is incorporated herein in its entirety.

### Example IV-1

### Synthesis of Compound IV-1

A mixture of compound **37a** (7.45 g, 27.79 mmol), *O*-methyl hydroxyl amine hydrochloride (2.75 g, 32.93 mmol), TBTU (11.4 g, 35.5 mmol) andNMM (10 mL) in dry DMF (20 mL) was stirred at room temperature overnight. Excess solvent was removed and the mixture was diluted with EtOAc that was washed successively with water, 2% citric acid, water, 2% NaHCO₃, water and brine. Drying (MgSO₄) and solvent evaporation gave a crude product that was purified by flash chromatography (silica gel, hexane : EtOAc 2 : 3) to generate 7.71 g of compound **IV-1;** ¹H-NMR (DMSO-d₆): δ 11.1 (s, 1H), 7.43 - 7.22 (series of m, 10H), 5.43 (s, 1H), 3.55 (s, 3H), 2.86 (s, 2H).

### Example IV-2

### Synthesis of Compound IV-2

Oxidation of the compound **IV-1** (7.6 g, 26.48 mmol) with hydrogen peroxide (1 equiv.) in AcOH (25 mL), as described in Example III-2, generated compound **IV-2** (5.98 g); mp: 140 - 141°C; ¹H-NMR (DMSO-d₆): δ 11.36 (s, 1H), 7.52 - 7.32 (series ofm, 10H), 5.38 (s, 1H), 3.56 (s, 3H), 3.36 (d, 1H), 3.04 (d, 1H). MS: 303.88 (M + H).

### Example IV-3

### Synthesis of compound IV-3

Utilizing compound **37a** and O-Ethyl hydroxyl amine hydrochloride in step 1, compound **IV-3** was prepared; mp: 65°C; ¹H-NMR(DMSO-d₆): δ 11.22 (s, 1H), 7.50 - 7.34 (series of m, 10H), 5.38 (s, 1H), 3.76 (m, 2H), 3.31 (d, 1H), 3.06 (d, 1H), 1.11 (m, 3H). MS: 317.92 (M + H).

### Example IV-4

### Synthesis of compound IV-4

Utilizing compound **37b** and *O*-methyl hydroxyl amine hydrochloride in step 1, compound **IV-4** was prepared; mp: 103-104 °C; ¹H-NMR (DMSO-d₆): δ 11.34 (s, 1H), 7.56-7.51 (m, 4H), 7.28-7.24 (m, 4H), 5.45 (s, 1H), 3.56 (s, 3H), 3.40 (d, J = 13.53 Hz, 1H), 2.99 (d, J = 13.55 Hz, 1H). MS: 361.89 (M + Na). 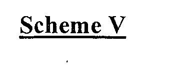 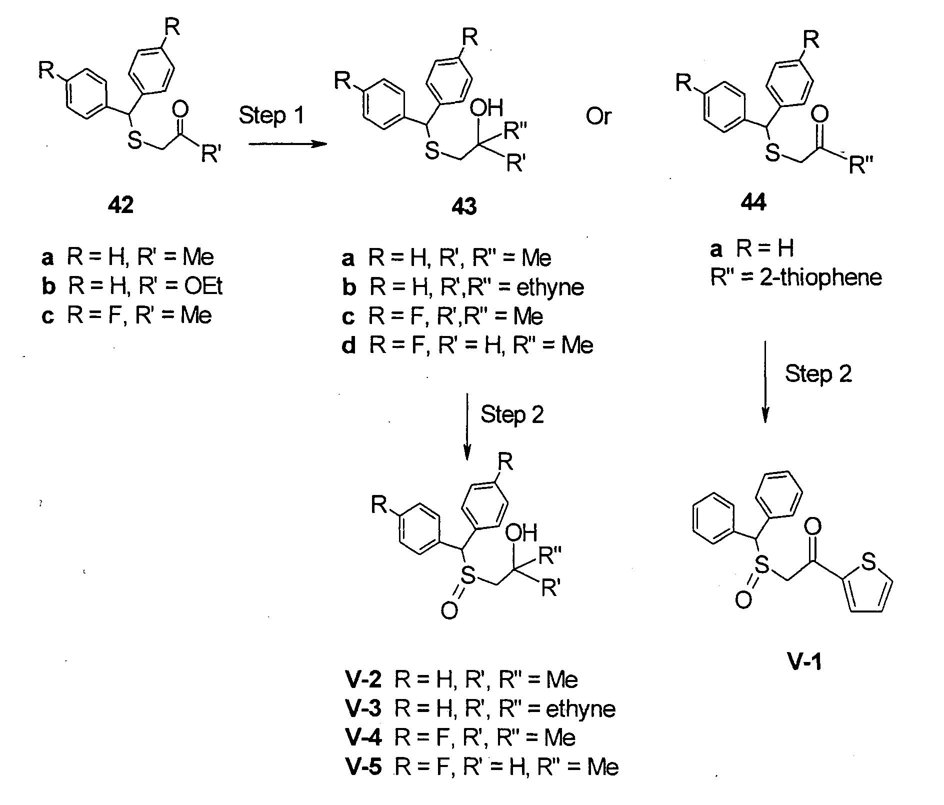
Reagents for Step 1: RMgBr or NaBH₄ (1.1 - 6 eqv.)/THF/0 °C - reflux.
Reagents for Step 2: 50% H₂O₂ in water/HOAc/RT

### Preparation of compound 42b

Compound **42b** was prepared as described in U.S. Pat. No. 4,006,686, which is incorporated herein in its entirety.

### Example V-1

### Synthesis of compound V-1

Utilizing compound **42b** and 2-thiophenylmagnesium bromide (1M in THF, 3.2 eqv.), compound **44a** was generated, which was then oxidized to give compound **V-1;** mp: 130 - 131 °C; ¹H-NMR (DMSO-d₆): δ 8.09 (d, 1H), 8.08 (d, 1H), 7.84-7.24 (series of m, 11H), 5.51 (s, 1H), 4.17 (s, 2H). MS: 340.84 (M+H)

### Example V-2

### Synthesis of the compound V-2

A solution of compound **42a** (the intermediate from the preparation of compound **III-3**, 6.4 g, 25 mmol) in anhydrous THF (60 mL) was added dropwise to a solution of methyl magnesium bromide (1.4 M in THF: toluene, 22 mL, 30.8 mmol) at 0 °C. The reaction mixture was stirred at 0 °C for an additional hour, quenched with saturated ammonium chloride solution, and extracted into EtOAc. The combined organic layers were washed successively with water and brine, dried (MgSO₄) and concentrated to generate compound **43a**. It was then taken in HOAc (25 mL) and oxidized with hydrogen peroxide (50% in water, 1.7 mL), as described before, to generate 4.31g of compound **V-2;** mp: 121 - 122 °C; ¹H-NMR (DMSO-d₆): δ 7.53 - 7.30 (series ofm, 10H), 5.22 (s, 1H), 4.86 (s, 1H), 2.71 (d, 1H), 2.44 (d, 1H), 1.18 (s, 3H), 1.13 (s, 3H). MS: 288.96 (M + H).

### Example V-3

### Synthesis of compound V-3

Utilizing compound **42b** and acetylenemagnesium bromide (0.5M in THF, 6 eqv) in step 1, compound **V-3** was generated; ¹H-NMR (DMSO-d₆): δ 7.54-7.31 (series of m, 10H), 6.99 (s, 1H), 5.32 (s, 1H), 3.69 (two s, 2H), 3.15 (d, 1H), 2.74 (d, 1H). MS: 308.88 (M+H),

### Example V-4

### Synthesis of compound V-4

Utilizing compound **42c** (the intermediate from Example III-12) and methyl magnesium bromide (3M in ether, 1.1 eqv), compound **V-4** (foam) was generated; ¹H-NMR (DMSO-d₆): δ 7.56 - 7.21 (two m, 8H), 5.31 (s, 1H), 4.87 (s, 1H), 2.71 (d, 1H), 2.39 (d, 1H), 1.29 (s, 3H), 1.14 (s, 3H). MS: 324.95 (M + H).

### Example V-5

### Synthesis of the compound V-5

To a solution of compound **42c** (4.85 g, 16.60 mmol, the intermediate from Example III-12) in methanol (85 mL) was added sodium borohydride (660 mg, 17.44 mmol) in portions. The reaction mixture was then stirred at room temperature for 0.5 h, quenched with ice-water and extracted into EtOAc. The combined organic layers were washed with brine, dried (magnesium sulfate), and concentrated to generate compound **43d** (oil, 4.62g) that was oxidized to generate compound **V-5** (mixture of diastereomers); mp: 96 - 98 °C, ¹H-NMR (DMSO-d₆): δ 7.56 - 7.21 (two m, 8H), 5.38 (s, 0.72H), 5.28 (s, 0.28H), 5.05 (m, 0.28H), 4.98 (m, 0.72H), 3.95(m, 1H), 2.50 (m, 2H), 1.13 (d, 2.16H), 1.09 (d, 0.84H). MS: 310.92 (M + H). 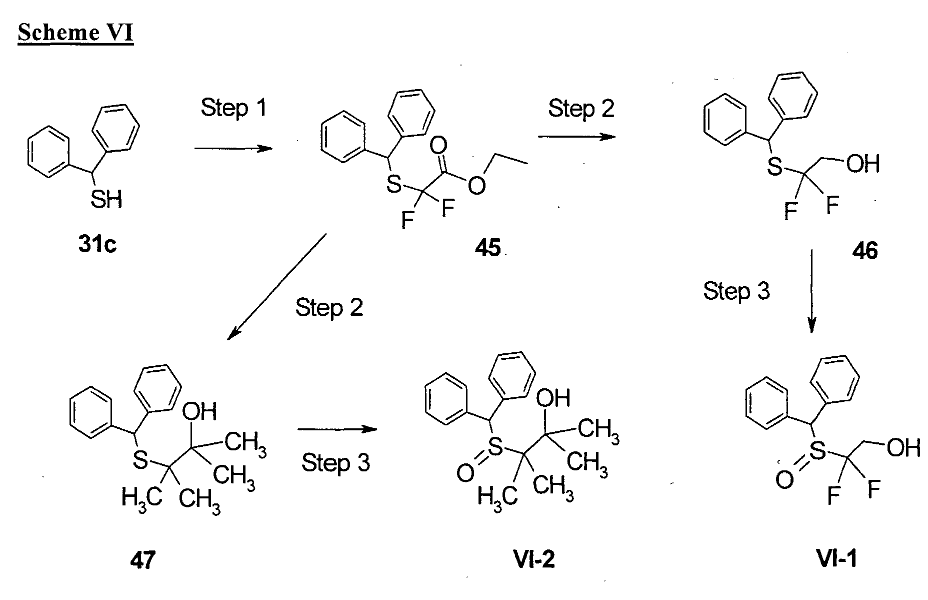
Reagents for Step 1: NaH/DMF/ethyl bromodifluoroacetate (compound **32**)/room temperature - 70 °C, 4 h.
Reagents for Step 2: Lithium aluminum hydride ("LAH")(1M in Et₂O)/THF/0 °C, 1.5h for compound **46;** MeMgBr for compound **47.**
Reagents for Step 3: *m*-Chloroperbenzoic acid ("m-CPBA"), dichloromethane, 0 °C.

### Preparation of compound 31c

The preparation of compound **31c** was described in U.S. Pat. No. 4,066,686, which has been incorporated herein in its entirety.

### Synthesis of the compound 46

Step 1: A solution of compound **31c** (9.24 g, 46.2 mmol) in dry DMF (3 mL) was slowly added to a stirred mixture ofNaH (60% in oil, 2.2 g, 55 mmol) in dry DMF (15 mL, room temperature, argon). The mixture was stirred at room temperature for 15 min, treated with compound **32** (11.41 g, 61 mmol) and heated at 70 °C for 4 h. The reaction mixture was then cooled to room temperature, concentrated at high vacuum, quenched with ice-water and extracted into EtOAc. The combined organic layers were washed successively with water and brine, dried (MgSO₄) and concentrated to generate compound **45** (12.34 g) that was directly used in the next step without any further purification.

Step 2: Thus, a solution of lithium aluminum hydride (1M in Et₂O) was slowly added to a solution of compound **45** (6.08 g, 18.88 mmol) in dry THF (50 mL, 0 °C, argon). The mixture was stirred at 0 °C for 1.5 h, treated successively (carefully) with EtOAc (5 mL), water (5 mL), and 10% H₂SO₄ (20 mL). The mixture was then extracted into EtOAc. The combined organic layers were washed successively with water (twice) and brine, dried (MgSO₄), and concentrated to give a crude product that was purified by flash chromatography (silica gel, hexane : EtOAc 4:1) to generate compound 46 (syrup, 2.87g); ¹H-NMR (DMSO-d₆): δ 7.47 - 7.22 (3 m, 10H), 5.86 (t, 1H), 5.78 (s, 1H), 3.70 (m, 2H).

### Example VI-1

### Synthesis of Compound VI-1

Following the procedure as described in Example II-19, compound **46** (2.68 g, 9.57 mmol) was oxidized with *m*-CPBA (77%, 2.36 g, 10.53 mmol) to generate compound **VI-1** (2.08 g), mp: 77 - 79 °C, ¹H-NMR (DMSO-d₆): δ 7.58 - 7.32 (two m, 10H), 6.05 (m, 1H), 5.68 (s, 1H), 3.99 - 3.70 (two m, 2H). MS: 318.96 (M+H).

### Example VI-2

### Synthesis of compound VI-2

Step 2: A solution of compound **45** (5.7 g, 17.7 mmol) in dry THF (40 mL) was added dropwise to a solution of methyl magnesium bromide (1.4 M in toluene, 65 mL, 91 mmol) under argon at room temperature. The reaction mixture was then stirred for 6 h, quenched with saturated ammonium chloride solution, and extracted into EtOAc. The combined organic layers were washed successively with water and brine, dried (MgSO₄) and concentrated to give a crude product that was purified by flash chromatography (silica gel, hexane: EtOAc 1 : 1) to provide compound **47** (766 mg) that was utilized in next step; ¹H-NMR (DMSO-d₆): δ 7.55 - 7.26 (series of m, 10H), 5.6 (s, 1H), 4.84 (s, 1H), 1.19 (two overlapping s, 6H), 1.09 (s, 3H), 0.9 (s, 3H).

Step 3: Thus, oxidation of compound **47** (0.76g, 2.53 mmol) with *m*-CPBA (77%, 0.625g, 2.78 mmol), following the procedure described in Example III-2, generated compound **VI-2** (0.291 g); mp: 103 -104 °C; ¹H-NMR (DMSO-d₆): δ 7.55-7.24 (two m, 10H), 5.60 (s, 1H), 4.84 (s, 1H), 1.19 (s, 6H), 1.09 (s, 3H), 0.90 (s, 3H). MS: 316.95 (M+H). 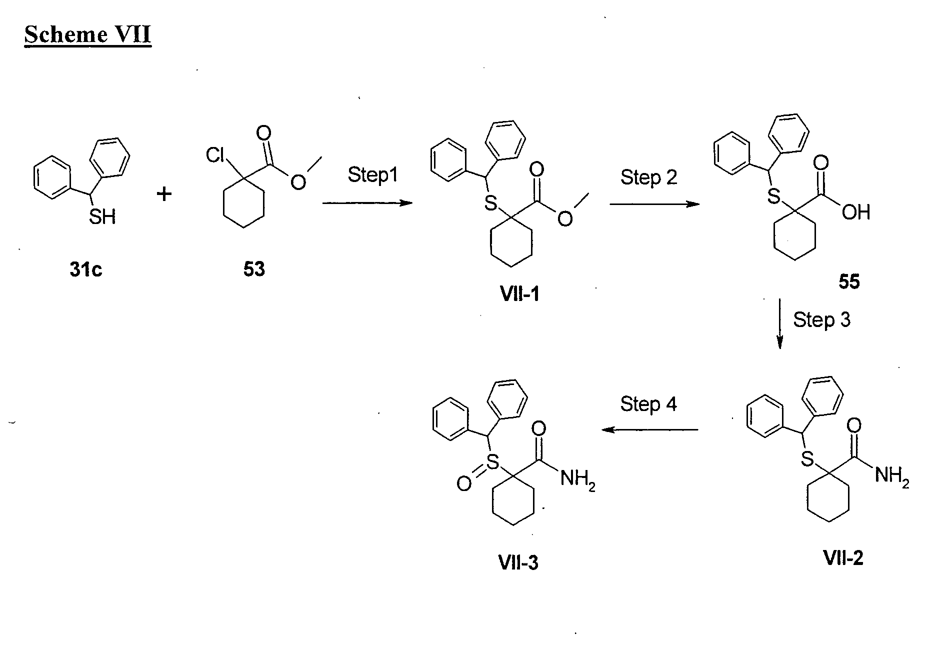
Reagents for Step 1: NaH/DMF/room temperature.
Reagents for Step 2: 1N NaOH/EtOH/reflux.
Reagents for Step 3: HOBT•NH₃/EDCI/DMF/room temperature.
Reagents for Step 4: 50% H₂O₂ in water/HOAc/room temperature.

### Example VII-1

### Synthesis of compound VII-1

A solution of compound **31c** (8.89 g, 44.45 mmol) in dry DMF (20 mL) was added dropwise to a stirred mixture ofNaH (60% in oil, 2.2 g, 55 mmol) in dry DMF (40 mL, under argon, room temperature). The mixture was stirred for 15 min, treated with compound 53 (8 mL, 50.3 mmol) and continued stirred overnight. Excess solvent was removed and the residue was quenched with water followed by extraction into EtOAc. The combined organic layers were washed successively with water (twice) and brine, dried (MgSO₄) and concentrated to give a residue that was purified by flash chromatography (silica gel, hexane : EtOAc 3 : 2) to generate compound **VII-1** (oil, 6.68 g); ¹H-NMR (DMSO-d₆): δ 7.44 - 7.20 (m, 10H), 5.21 (m, 10H), 5.21 (s, 1H), 3.6 (s, 3H), 2.7 (m, 1H), 2.16 (m, 1H), 1.98-1.15 (series of m, 8H).

### Example VII-2

### Synthesis of compound VII-2

A mixture of the compound **VII-1** (6.68 g, 19.64 mmol), NaOH (1N, 100 mL) and EtOH (100 mL) was kept under reflux for 3h. The mixture was cooled to room temperature, concentrated and washed with ether. The basic aqueous layer was neutralized with conc. HCl and extracted into EtOAc. The combined organic layers were washed successively with water (twice) and brine, dried (MgSO₄), and concentrated to yield compound 55 that was directly used in the next step without further purification.

Thus, a mixture of compound **55** (4.96 g, 15.21 mmol), HOBT•NH₃ complex (5 g, 32.89 mmol followed by an additional amount of 2.5 g, 16.44 mmol after 2 h), EDCI (3.5g, 18.3 mmol followed by an additional amount of 1.75g, 9.1 mmol after 3h) in DMF (50 mL) was stirred at room temperature overnight, diluted with dichloromethane, washed successively with water, 2% citric acid, water, 2% NaHCO₃, water and brine, and dried (MgSO₄). Solvent evaporation generated a crude product that was purified by flash chromatography (silica gel, hexane:EtOAc :: 1:2) to yield compound **VII-2** (2.42 g); ¹H-NMR (DMSO-d₆): δ 7.44-7.06 (series of m, 10H), 6.98 (br s, 2H), 5.30 (s, 1H), 3.03 (m, 1H), 2.43 (m, 1H), 1.83-1.11 (series of m, 8H).

### Example VII-3

### Synthesis of compound VII-3

To a solution of the compound **VII-2** (2.2 g, 6.76 mmol) in AcOH (10 mL) was added hydrogen peroxide (50% solution in water, 470 µL). The reaction mixture was stirred at room temperature for 5 h, filtered and concentrated to give a crude product that was purified by flash chromatography (silica, EtOAc) to generate compound **VII-3** (0.593 g) mp: 155 - 156 °C, ¹H-NMR (DMSO-d₆): δ 7.55 - 7.30 (series ofm, 11H), 7.07 (s, 1H), 5.14 (s, 1H), 2.73 (m, 1H), 2.63 (m, 1H), 1.82 - 1.19 (series of m, 8H). MS: 342 (M + Na). 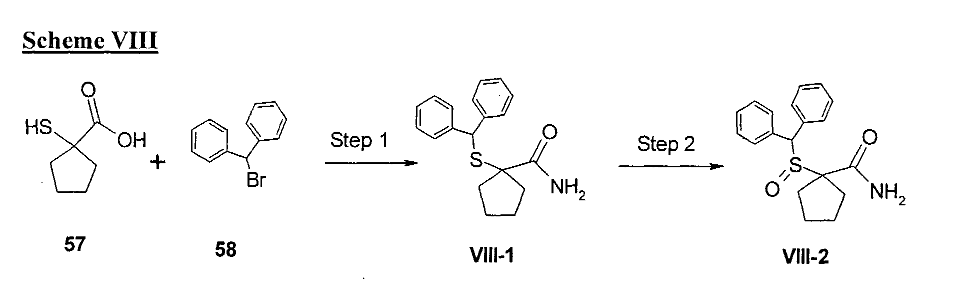
Reagents for Step 1: a) 100 °C; b): HOBT•NH₃/TBTU/DMF/room temperature.
Reagents for Step 2: 50% H₂O₂ in water/HOAc/0 °C to room temperature.

### Preparation of compound 57

The preparation of compound **57** was described by Seebach, D.; Teschner, M. *Chem. Ber*. **1976**, *109*, 1601, which is incorporated herein in its entirety.

### Example VIII-1

### Synthesis of compound VIII-1

A mixture of compound **57** (116 mg, 0.79 mmol and 304 mg, 2.08 mmol, respectively, in two batches) and compound **58** (1 eqv. in each case) was heated at 100 °C for 1 h and cooled to room temperature to give an adduct that was subjected to subsequent amidation as described in Scheme VII, Step 3, to generate compound **VIII-1** (0.346 g from two batches); ¹H-NMR (DMSO-d₆): δ 7.49 - 7.27 (series ofm, 11H), 7.04 (s, 1H), 5.23 (s, 1H), 2.02 -1.35 (3 m, 8H).

### Example VIII-2

### Synthesis of the Compound VIII-2

Following the procedure described in Example III-2, compound **VIII-1** (342 mg, 1.09 mmol) was oxidized with hydrogen peroxide (75 µL) in AcOH (5 mL) to generate compound **VIII-2** (0.282 g); mp: 129 - 130 °C; ¹H-NMR (DMSO-d₆): 7.47 - 7.28 (m, 11H), 7.15 (s, 1H), 5.13 (s, 1H), 1.93 - 1.26 (3m, 8H). MS: 328 (M + H) 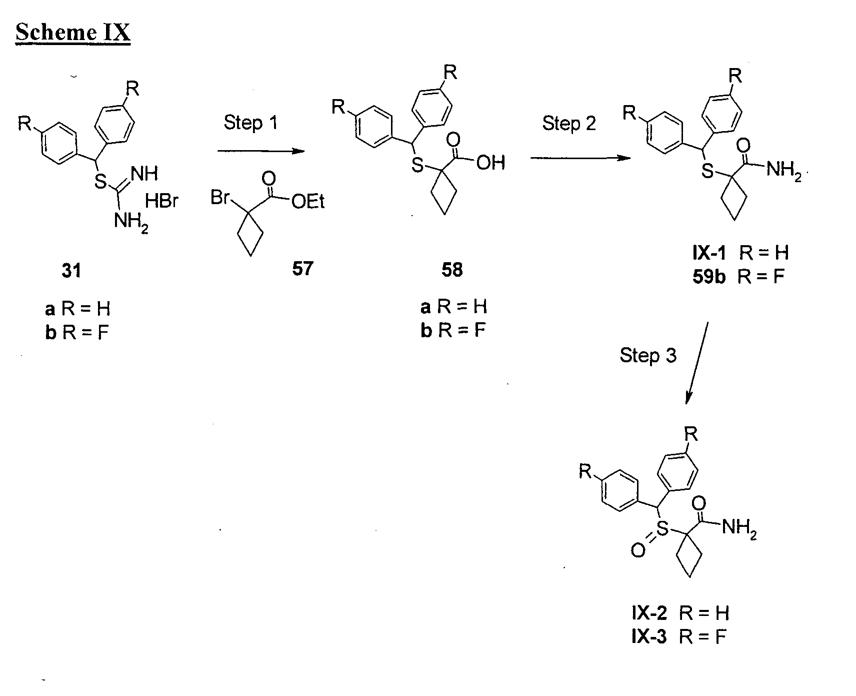
Reagents for Step 1: a) 10 N NaOH/EtOH/70 °C; b) compound 57, 70 °C, overnight.
Reagents for Step 2: HOBT•NH₃/TBTU/NMM/DMF/room temperature.
Reagents for Step 3: 50% H₂O₂ in water/HOAc/room temperature.

### Example IX-1

### Synthesis of the Compound IX-1

A mixture of compound **31a** (2 g, 6.19 mmol), 10 N NaOH (3 mL, 30 mmol) and water (3 mL) was stirred under argon at 70 °C for 15 min, treated with compound **57** (1 mL, 6.17 mmol) followed by heating at 70 °C overnight, and cooled to room temperature. It was then extracted into ether and the combined organic layers were washed successively with water and brine, dried (MgSO₄) and concentrated to generate compound **58a** (1.46 g) that was directly taken to next step without further purifications. Thus, a mixture of compound **58a** (1.45 g, 4.8 mmol), HOBT•NH₃ complex (1.63 g, 10.72 mmol), TBTU (1.87g, 5.8 mmol), and NMM (2 mL) in dry DMF (10 mL) was stirred at room temperature overnight, diluted with EtOAc and washed successively with water, 2% citric acid, water, 2% NaHCO₃, water and brine. Drying (MgSO₄) and solvent evaporation generated a crude product that was purified by flash chromatography (silica gel, hexane : EtOAc 3 : 7) to yield compound **IX-1** (0.395g); ¹H-NMR (DMSO-d₆): δ 7.42 - 7.20 (3 m, 10H), 6.97 (two overlapping broad s, 2H), 5.37 (s, 1H), 2.62 (s, 2H), 0.97 (m, 2H), 0.53 (m, 2H).

### Example IX-2

### Synthesis of Compound IX-2

Following the procedure described in Example III-2, compound **IX-1** (384 mg, 1.29 mmol) was oxidized with hydrogen peroxide (90 µL) in AcOH (3 mL) to generate compound **IX-2** (0.342 g); mp 158 - 159 °C; ¹H-NMR (DMSO-d₆): δ 7.5 - 7.3 (m, 10H), 6.9 (two broad overlapping s, 2H), 5.23 (s, 1H), 3.13 (d, 1H), 2.31 (d, 1H), 1.23 - 0.62 (4 m, 4H). MS: 336 (M + Na).

### Example IX-3

### Synthesis of compound IX-3

Utilizing compound **31b** and compound **57** in step 1 and following the procedure as described above, compound **IX-3** was generated; mp: 162 °C; ¹H-NMR (DMSO-d₆): δ 7.54 - 7.50 (m, 4H), 7.26 - 7.21 (m, 4H), 6.97 (two overlapping broad s, 2H), 5.32 (s, 1H), 3.2 (d, 1H), 2.19 (d, 1H), 1.20 - 0.69 (4 m, 4H). MS: 350 (M + H) 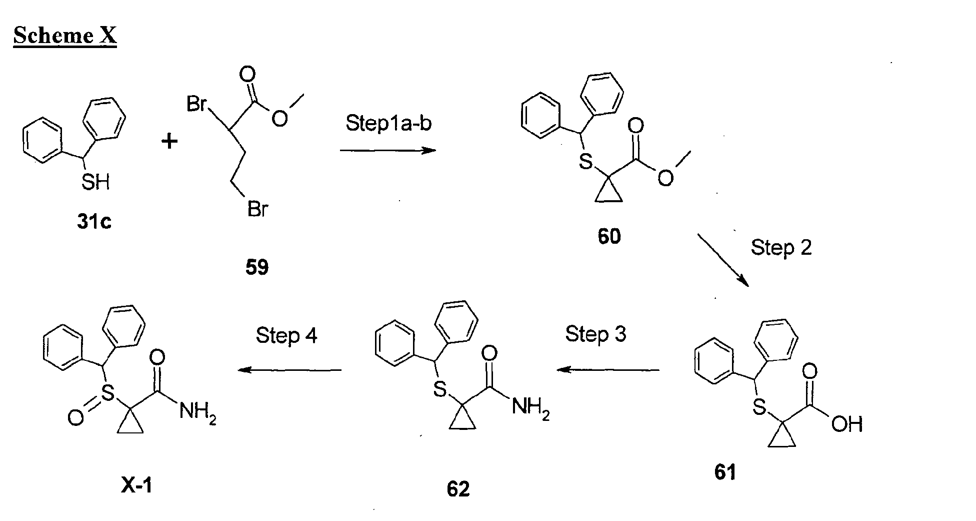
Reagents in Step 1: a) K₂CO₃/toluene/room temperature to 80°C. b) K₂CO₃/Bu₄NHSO₄/Toluene/80 °C.
Reagents in Step 2: 1N NaOH/MeOH/reflux.
Reagents in Step 3: HOBT•NH₃/EDCI/DMF/RT.
Reagents in Step 4: 50% H₂O₂ in water/HOAc/room temperature.

### Preparation of compound 59

Preparation of compound **59** was described by Hoffmann, H. M. R.; Eggert, U.; Walenta, A.; Weineck, E.; Schomburg, D.; Wartchow, R.; Allen, F. H. *J. Org. Chem*. **1989,** *54*, 6096, which is incorporated herein by reference in its entirety.

### Preparation of compound 60

A mixture of the compound **31c** (3.66 g, 18.3 mmol), compound **59** (6.3 g, 24.23 mmol), anhydrous K₂CO₃ (7.5 g, 54.34 mmol) in anhydrous toluene (50 mL) was stirred at room temperature for 96 h and then at 80 °C for 3h. n-Bu₄NHSO₄ (450 mg) was added to the reaction mixture and stirring was continued at 80 °C for another 72 h. Additional quantities of K₂CO₃ (3 g), Bu₄NHSO₄ (150 mg), and toluene (20 mL) were then added to the reaction mixture and heating (at 80 °C) was continued for another 96 h. After cooling to room temperature, the reaction mixture was filtered and residue was washed with diethyl ether. The combined filtrate and washings were concentrated to give the crude product, **60**, (5.17g) which was directly used for the next step without further purifications.

### Preparation of compound 62

A mixture of compound **60** (5.15 g, 17.28 mmol), NaOH (1N, 100 mL), methanol (100 mL) was kept under reflux for 4 h, cooled to room temperature, and concentrated to remove excess methanol. Basic layer was acidified with conc. HCl and extracted into EtOAc. The combined organic layers were washed with brine, dried (MgSO₄), and concentrated to generate compound **61** (4.45 g) that was directly used in the next step.

Thus, a mixture of compound **61** (4.43 g, 15.65 mmol), HOBT•NH₃ complex (5.3 g, 34.86 mmol), EDCI (3.5 g, 18.3 mmol), DMAP (380 mg) in DMF (30 mL) was stirred at room temperature overnight, diluted with dichloromethane, successively washed successively with water, 2% citric acid, water, 2% NaHCO₃, water and brine, and dried (MgSO₄). Solvent evaporation gave a crude product that was purified by flash chromatography (silica gel, hexane : EtOAc 1:1) to generate the product (2.38g); ¹H-NMR (DMSO-d₆): δ 7.45 - 7.21 (3 m, 12H), 5.37 (s, 1H), 1.76 (m, 2H), 0.82 (m, 2H).

### Example X-1

### Synthesis of Compound X-1

Compound **62** (2.34 g, 8.26 mmol) was oxidized with hydrogen peroxide (570 µL) in AcOH (12 mL) to generate compound **X-1** (1.81 g); mp: 148 - 149 °C, ¹H-NMR (DMSO-d₆): δ7.5 - 7.31 (m, 11H), 7.25 (s, 1H), 5.51 (s, 1H), 1.0 (m, 2H), 0.63 (m, 2H). MS: 322 (M + Na).

### Utility

The present invention provides a method of treating diseases and conditions in a subject in need thereof comprising administering to said subject a therapeutically effective amount of a compound of the present invention. For example, the compounds of the present invention may be useful for the treatment of diseases, such as excessive sleepiness, promotion and/or improvement of wakefulness (preferably improvement of wakefulness in patients with excessive sleepiness associated with narcolepsy, sleep apnea (preferably obstructive sleep apnea/hypopnea) and shift work disorder), treatment of Parkinson's disease, Alzheimer's disease, cerebral ischemia, stroke, eating disorders, attention deficit disorder ("ADD"), attention deficit hyperactivity disorder ("ADHD"), depression, schizophrenia, fatigue (preferably fatigue associated with cancer or neurological diseases, such as multiple sclerosis and chronic fatigue syndrome), stimulation of appetite and weight gain and improvement of cognitive dysfunction.

### Methodology: Evaluation of Wake Promoting Activity in Rats

The methodology utilized for evaluating wake promoting activity of test compounds is based on that described by Edgar and Seidel, *Journal of Pharmacology and Experimental Therapeutics,* **283**:757-769, 1997, and incorporated herein in its entirety by reference.

**Animal Surgery.** Adult, male Wistar rats (275-320g from Charles River Laboratories, Wilmington, MA) were anesthetized (Nembutal, 45 mg/kg, ip.) and surgically prepared with implants for recording of chronic EEG (encephalographic) and EMG (electromyographic) recording. The EEG implants were made from commercially available components (Plastics One, Roanoke, VA). EEG signals were recorded from stainless steel screw electrodes: 2 frontal (+3.0 mm AP from bregma, ±2.0 mm ML), and 2 occipital (-4.0 mm AP from bregma, ±2.0 mm ML). Two Teflon-coated stainless steel wires were positioned under the nuchal trapezoid muscles for EMG recording. All electrode leads were inserted into a connector pedestal and the pedestal affixed to the skull by application dental acrylic. Antibiotic was administered post surgically and antibiotic cream was applied to the wound edges to prevent infection. At least one week elapsed between surgery and recording.

**Recording environment.** Postsurgically, rats were housed in pairs in an isolated room. Food and water were available *ad libitum,* ambient temperature was 21°C, and humidity was 55%. At least 24 hrs prior to recording, they were placed in Nalgene containers (31 x 31 x 31 cm) with a wire-grid top, and entry to the room was prohibited during the day of recording except for dosing. The containers were placed on a rack with two shelves, 4 containers per shelf. Fluorescent overhead room lights were set to a 24 hr. light/dark cycle (on at 7 AM, off at 7 PM). Light levels inside the containers were 38 and 25 lux for the top and bottom shelves respectively. Background white-noise (68db inside the containers) was present in the room to mask ambient sounds.

**Data acquisition**. EEG and EMG signals were led via cables to a commutator (Plastics One) and then to pre-amplifiers (model 1700, A-M Systems, Carlsborg, WA). EEG and EMG signals were amplified (10K and 1K respectively) and band pass filtered between 0.3 and 500 Hz for EEG and between 10 and 500 Hz for EMG. These signals were digitized at 128 samples per second using ICELUS sleep research software (M. Opp, U. Texas; see Opp, Physiology and Behavior 63:67-74, 1998, and Imeri, Mancia, and Opp, *Neuroscience* **92**:745-749, 1999, incorporated by reference herein in their entirety) running under Labview 5.1 software and data acquisition hardware (PCI-MIO-16E-4; National Instruments, Austin, TX). On the day of dosing, data was recorded for 6 to 10 hours beginning at 11 AM.

**Drug administration and study design**. Compounds were evaluated on groups of from 4 to 8 rats carried out over one or two separate test sessions. Each animal was tested with a different compound or vehicle for up to 10 weeks with at least 7 days between successive tests. A vehicle group was included in all experiments, and each animal received vehicle every 4^{th} test. Test compounds were suspended in sterile 0.25% methylcellulose (pH=6.2; Upjohn Co., Kalamazoo, MI) at 30 mg/mL. Unless otherwise noted, compounds were administered at a single dose of 100 mg/kg. Dosing was carried out at noon, while the rats were predominantly asleep. Each rat was lifted out of its container, given an intraperitoneal injection in a volume of 5 mL/kg, and replaced. Dosing required approximately 30 sec per rat.

**Sleep / wake scoring.** Sleep and wake activity were determined using a procedure involving manual scoring using the ICELUS software, followed by application of an autoscoring program written in Microsoft Excel (Microsoft, Inc., Redmond, WA) The ICELUS program displays the EEG and EMG data in blocks of 6 sec along with the EEG frequency spectrum (FFT) amplitudes. Arousal state was scored as awake, rapid eye-movement (REM), or slow-wave or non-REM sleep according to visual analysis of EEG frequency and amplitude characteristics and EMG activity (Opp and Krueger, 1994; Van Gelder, *et al*., 1991; Edgar, *et al*., 1991, 1997; Seidel, *et al*, 1995, incorporated by reference herein in their entirety). Essentially, waking activity consists of relatively low-amplitude EEG activity with relatively lower power in the frequency band from 0.5 - 6 Hz, accompanied by moderate to high level EMG activity. In a particular waking state ("theta-waking"), EEG power can be relatively focused in the 6-9 Hz (theta) range, but significant EMG activity is always present. NREM sleep is characterized by relative high-amplitude EEG activity with relatively greater power in the low frequency band from 0.5 - 6 Hz, accompanied by little or no EMG activity. REM sleep is characterized by moderate and constant amplitude EEG focused in the theta (6-9 Hz) range, similar to waking theta, but with no EMG activity.

To convert the raw data to sleep / wake stage scores, normally the first hour of activity (prior to dosing) is manually scored into sleep, wake, or REM states. Subsequent activity is evaluated using a computer algorithm which takes into account FFT amplitudes, theta-band activity, and EMG activity for each 6 second epoch. An iterative procedure is used to adjust 3 different parameter thresholds until the first hour of data scored by the computer algorithm matches as closely as possible with the manual values. These parameter values are then used to score the remaining activity. The data are then reduced to "wake" (wake + waking theta activity) or "sleep" (REM + non-REM) for each 6 sec epoch. The time spent awake was then calculated for each 5 and 30 min interval relative to the specific time of dosing (approximately 12:00 noon).

### Data analysis and statistics.

Two basic outcome measures were used to ascertain whether a compound exhibited wake-enhancing activity. The first was the percent time spent awake (0 - 100%) for each 30 min period following dosing. The second was the sum in minutes of the time spent awake for the first 6 half-hour periods following dosing (3 hr AUC; maximum 180 min).

For purposes of ascertaining activity of a test compound, wake activity values were compared against corresponding vehicle values. The vehicle values were of two types. The first type was the corresponding within-experiment vehicle, that is, a value derived from the vehicle group run concurrently with the test compound. A second reference vehicle value was also used for comparison, which consisted of the mean 3 hr AUC value calculated from 234 animals in 59 separate experiments carried out during the same time period as the evaluations of the test compounds (mean ± SD = 69.22 ± 20.12; 95% confidence limits = 66.63 - 71.81). Two-tailed, unpaired t-tests were performed on the wake time values for drug versus vehicle treated animals, and compounds with p ≤ 0.05 were deemed significantly wake-promoting. A test compound was considered active as a wake promoting agent if it met one or more of the following three criteria.
(i) The 3 hr AUC value for the test compound was significantly greater (p ≤ 0.05) than the mean wake value for the reference vehicle group (N = 234).
(ii) The 3 hr AUC value for the test compound was significantly greater (p ≤ 0.05) than the corresponding value for the within -experiment vehicle group.
(iii) One or more of the half-hour wake time values from 0.5 to 2 hrs after dosing were significantly greater (p ≤ 0.05) in the test compound group compared to the within-experiment vehicle group.

### Results.

Compounds of the invention either have demonstrated or are expected to demonstrate utility for wake promoting activity.

**References.** The following references, to the extent that they provide exemplary procedural or other details supplementary to those set forth herein, are specifically incorporated in their entirety herein by reference:
Touret, *et al*., *Neuroscience Letters,* **189**:43-46, 1995.
Van Gelder, R.N. *et al*., *Sleep* **14**:48-55, 1991.
Edgar, D.M., *J. Pharmacol. Exp. Ther.* **282**:420-429, 1997.
Edgar and Seidel, *J. Pharmacol. Exp. Ther.,* **283**:757-69, 1997.
Hernant *et al*., *Psychopharmacology,* **103**:28-32, 1991.
Lin *et al*., *Brain Research,* **591**:319-326, 1992.
Opp and Krueger, *American Journal of Physiology* **266**:R688-95, 1994
Panckeri *et al*., *Sleep,* **19**(8):626-631, 1996.
Seidel, W.F., *et al*., *J. Pharmacol. Exp. Ther.* **275**:263-273, 1995.
Shelton *et al*., *Sleep* **18**(10):817-826, 1995.
Welsh, D.K., *et al*., *Physiol. Behav.* **35**:533-538, 1985.

### Dosage and Formulation.

The compounds of the present invention can be administered for therapeutic purposes by any means that results in the contact of the active agent with the agent's site of action in a subject. The compounds may be administered by any conventional means available for use in conjunction with pharmaceuticals, either as individual therapeutic agents or in a combination with other therapeutic agents, such as, for example, analgesics, or in combination with antidepressants, including but are not limited to tricyclic antidepressants ("TCAs"), Selective Serotonin Reuptake Inhibitors ("SSRIs"), Serotonin and Noradrenalin Reuptake Inhibitors ("SNRIs"), Dopamine Reuptake Inhibitors ("DRIs"), Noradrenalin Reuptake Inhibitors ("NRUs"), Dopamine, Serotonin and Noradrenalin Reuptake Inhibitors ("DSNRIs") and Monoamine Oxidase Inhibitors ("MAOIs) including reversible inhibitors of monoamine oxidase type A (RIMAs). The compounds of the present invention are preferably administered in therapeutically effective amounts for the treatment of the diseases and disorders described herein.

A therapeutically effective amount can be readily determined by the attending diagnostician, as one skilled in the art, by the use of conventional techniques. The effective dose will vary depending upon a number of factors, including the pharmacodynamics of the active agent, the type and extent of progression of the disease or disorder, the age, weight and health of the particular patient, the formulation of the active and its mode and frequency of administration, and the desired effect with a minimization of side effects. Typically, the compounds are administered at lower dosage levels, with a gradual increase until the desired effect is achieved.

Typical dose ranges are from about 0.01 mg/kg to about 100 mg/kg of body weight per day, with a preferred dose from about 0.01 mg/kg to 10 mg/kg of body weight per day. A typical daily dose for adult humans can range from about 1 to about 1000 mg of the active agent, particularly from about 1 to about 400 mg, and including 25, 50, 85, 100, 150, 170, 200, 255, 250, 255, 340, 400, 425, 500, 600, 700, 750, 800, and 900 mg doses, and equivalent doses for a human child.

The compounds may be administered in one or more unit dose forms, and they may be administered in a single daily dose or in two, three or four doses per day. The unit dose ranges from about 1 to about 1000 mg, particularly from about 1 to about 400 mg, and including 25, 50, 85, 100, 150, 170, 200, 255, 250, 255, 340, 400, 425, 500, 600, 700, 750, 800, and 900 mg unit doses, and equivalent unit doses for a human child. In particular, the unit dosages range from about 1 to about 500 mg administered one to four times a day, preferably from about 10 mg to about 300 mg, two times a day. In an alternate method of describing an effective dose, an oral unit dose is one that is necessary to achieve a blood serum level of about 0.05 to 20 µg/ml in a subject, and preferably about 1 to 20 µg/ml.

The compounds of the present invention may be formulated into pharmaceutical compositions by admixture with one or more pharmaceutically acceptable excipients. The active agent may be present in about 0.5-95% by weight of the composition. The excipients are selected on the basis of the chosen route of administration and standard pharmaceutical practice, as described, for example, in *Remington: The Science and Practice of Pharmacy*, 20^{th} ed.; Gennaro, A. R., Ed.; Lippincott Williams & Wilkins: Philadelphia, PA, 2000.

The compositions can be prepared for administration by oral means, including tablets, pills, powders, capsules, troches and the like; parenteral means, including intravenous, intramuscular, and subcutaneous means; topical or transdermal means, including patches, creams, ointments, lotions, pastes, gels, solutions, suspensions, aerosols, and powders and the like; transmucosal means, including nasal, rectal, vaginal, sublingual and buccal means; ophthalmic or inhalation means. Preferably the compositions are prepared for oral administration, particularly in the form of tablets, capsules or syrups; parenteral administration, particularly in the form of liquid solutions, suspensions or emulsions; intranasal administration, particularly in the form of powders, nasal drops, or aerosols; or for topical use, such as patches, creams, ointments, and lotions.

For oral administration, the tablets, pills, powders, capsules, troches and the like can contain one or more of the following: diluents or fillers such as starch, or cellulose; binders such as microcrystalline cellulose, gelatins, or polyvinylpyrrolidone; disintegrants such as starch or cellulose derivatives; lubricants such as talc or magnesium stearate; glidants such as colloidal silicon dioxide; sweetening agents such as sucrose or saccharin; and flavoring agents such as peppermint or cherry flavoring. Capsules may contain any of the above ingredients, and may also contain a semi-solid or liquid carrier, such as a polyethylene glycol. The solid oral dosage forms may have coatings of sugar, shellac, or enteric agents. Liquid preparations may be in the form of aqueous or oily suspensions, solutions, emulsions, syrups, elixirs, etc., or may be provided as a dry product for reconstitution with water or other suitable vehicle before use. Such liquid preparations may contain conventional additives such as surfactants, suspending agents, emulsifying agents, diluents, sweetening and flavoring agents, dyes and preservatives.

The compositions may also be administered parenterally. The pharmaceutical forms acceptable for injectable use include, for example, sterile aqueous solutions, or suspensions. Aqueous carriers include mixtures of alcohols and water, buffered media, and the like. Nonaqueous solvents include alcohols and glycols, such as ethanol, and polyethylene glycols; oils, such as vegetable oils; fatty acids and fatty acid esters, and the like. Other components can be added including surfactants; such as hydroxypropylcellulose; isotonic agents, such as sodium chloride; fluid and nutrient replenishers; electrolyte replenishers; agents which control the release of the active compounds, such as aluminum monostearate, and various co-polymers; antibacterial agents, such as chlorobutanol, or phenol; buffers; suspending agents; thickening agents; and the like. The parenteral preparations can be enclosed in ampules, disposable syringes or multiple dose vials. Other potentially useful parenteral delivery systems for the active compounds include ethylene-vinyl acetate copolymer particles, osmotic pumps, implantable infusion systems, and liposomes.

Other possible modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for topical use are in the form of an ointment, cream, or gel. Typically these forms include a carrier, such as petrolatum, lanolin, stearyl alcohol, polyethylene glycols, or their combinations, and either an emulsifying agent, such as sodium lauryl sulfate, or a gelling agent, such as tragacanth. Formulations suitable for transdermal administration can be provided as discrete patches, as in a reservoir or microreservoir system, adhesive diffusion-controlled system or a matrix dispersion-type system. Formulations for buccal administration include, for example lozenges or pastilles and may also include a flavored base, such as sucrose or acacia, and other excipients such as glycocholate. Formulations suitable for rectal administration are preferably provided as unit-dose suppositories, with a solid based carrier, such as cocoa butter, and may include a salicylate.

The compositions of the present invention may be formulated to control and/or delay the release of the active agent(s). Such controlled-, delayed, sustained-, or extended-release compositions are well-known in the art, and may include, for example, reservoir or matrix diffusion products, as well as dissolution systems. Some compositions may utilize, for example biocompatible, biodegradable lactide polymer, lactide/glycolide copolymer, or polyoxyethylene-polyoxypropylene copolymers as excipients.

As those skilled in the art will appreciate, numerous modifications and variations of the present invention are possible in light of the above teachings. It is therefore understood that within the scope of the appended claims, the invention may be practiced otherwise than as specifically described herein, and the scope of the invention is intended to encompass all such variations.

## Claims

1. A compound of formula (I): 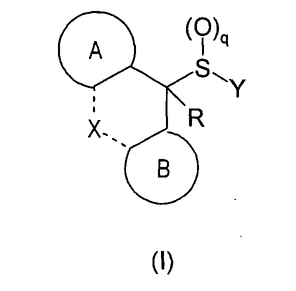 wherein
rings A and B, together with the carbon atoms to which they are attached, are each independently selected from:
a) a 6-membered aromatic carbocyclic ring in which from 1 to 3 carbon atoms may be replaced by hetero atoms selected from oxygen, nitrogen and sulfur; and
b) a 5-membered aromatic carbocyclic ring in which either:
i) one carbon atom may be replaced with an oxygen, nitrogen, or sulfur atom;
ii) two carbon atoms may be replaced with a sulfur and a nitrogen atom, an oxygen and a nitrogen atom, or two nitrogen atoms; or
iii) three carbon atoms may be replaced with three nitrogen atoms, one oxygen and two nitrogen atoms, or one sulfur and two nitrogen atoms;
wherein said rings are optionally substituted with one to three R²⁰ groups;
X is not present, is a bond, O, S(O)_{y}, NR¹⁰, C₂ alkylene, C₂₋₃ alkenylene, C(=O), C(R²¹)₂NR¹⁰, C(R²¹)=N, N=C(R²¹), C(=O)N(R¹⁰), or NR¹⁰C(=O); wherein said alkylene and alkenylene groups are optionally substituted with one to three R²⁰ groups;
R is H, C₁-C₆ alkyl;
Y is selected from:
a) C₁-C₆ alkylene-R¹;
b) C₁-C₆ alkylene-R²;
c) (C₁-C₄ alkylene)ₘ-Z-(C₁-C₄ alkylene)ₙ-R¹;
d) C₁-C₆ alkylene-O(CH₂)ₚOR²¹,
e) C₁-C₆ alkyl substituted with one or two OR¹¹ groups; provided that Y cannot be (CH₂)₁₋₄OR¹¹; and
f) CH₂CR²¹=C(R²¹)₂ except when X is a bond and q is 2;
wherein said alkyl and alkylene groups are optionally substituted with one to three R²⁰ groups;
Z is O, NR^{10A}, S(O)_{y}, CR²¹=CR²¹, C=C(R²¹)₂, C=C, C₆-C₁₀ arylene, 5-10 membered heteroarylene, C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
R¹ is selected from NR¹²R¹³, NR²¹C(=O)R¹⁴, C(=O)R¹⁵, CO₂R¹¹, OC(=O)R¹¹, C(=O)NR¹²R¹³, C(=O)NR²¹OR¹⁴, C(=NR¹¹)NR¹²R¹³, NR²¹S(O)₂R¹¹, S(O)₂NR¹²R¹³ NR²¹S(O)₂NR¹²R¹³, and PO(OR²¹)₂;
R² is a 5-6 membered heteroaryl, wherein said heteroaryl group is optionally substituted with one to three R²⁰ groups;
R¹⁰ and R^{10A} at each occurrence are independently selected from H, C₁-C₆ alkyl, C₆-C₁₀ aryl, C(=O)R¹⁵, and S(O)_{y}R¹⁴; wherein said alkyl and aryl groups are optionally substituted with one to three R²⁰ groups;
R¹¹ at each occurrence is independently selected from H and C₁-C₆ alkyl; wherein said alkyl is optionally substituted with one to three R²⁰ groups;
R¹² and R¹³ at each occurrence are each independently selected from H, C₁-C₆ alkyl, and C₆-C₁₀ aryl, or R¹² and R¹³, together with the nitrogen to which they are attached, form a 3-7 membered heterocycloalkyl ring;
wherein said alkyl and aryl groups and heterocycloalkyl ring are optionally substituted with one to three R²⁰ groups;
R¹⁴ at each occurrence is independently selected from C₁-C₆ alkyl, C₆-C₁₀ aryl, and arylalkyl; wherein said alkyl, aryl and arylalkyl groups are optionally substituted with one to three R²⁰ groups;
R¹⁵ at each occurrence is independently selected from C₁-C₆ alkyl, C₆-C₁₀ aryl, arylalkyl, and heteroaryl; wherein said alkyl, aryl, arylalkyl, and heteroaryl groups are optionally substituted with one to three R²⁰ groups;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocycloalkyl ring;
R²⁵ at each occurrence is independently the residue of an amino acid after the hydroxyl group of the carboxyl group is removed;
m is 0 or 1;
n is 0 or 1;
p is 1, 2, 3, or 4;
q is 0, 1, or 2;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
2) when X is not present, then R¹ cannot be NR¹²R¹³;
and the stereoisomeric forms, mixtures of stereoisomeric forms or pharmaceutically acceptable salts forms thereof.

2. The compound of claim 1 having a structure of formula (IV): 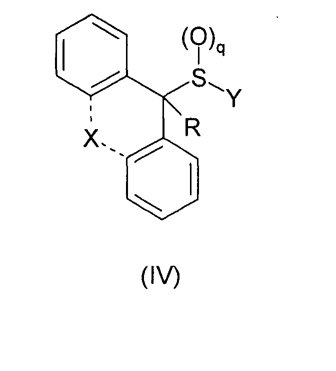 wherein
the phenylene rings are each independently optionally substituted with one to three R²⁰ groups;
X is not present or is a bond;
R is H or C₁-C₄ alkyl;
Y is selected from:
a) C₁-C₆ alkylene-R¹;
b) C₁-C₆ alkylene-R²;
c) C₁-C₄ alkylene-O(CH₂)ₚOR²¹,
d) CH₂C(OH)(CH₃)₂, CH₂C(CH₃)₂OH, CH₂C(OH)₂CF₃, CH₂C(OH)(C≡CH)₂, or CH₂CH(OH)CH₃,
e) CH₂CR²¹=C(R²¹)₂ CH₂CR²¹=C(R²¹)₂ except when X is a bond and q is 2;
wherein said alkylene groups optionally substituted with an R²⁰ group;
R¹ is selected from pyrrolidinyl, piperidinyl, morpholinyl, NR²¹C(=O)R¹⁴, C(=O)R¹⁵, CO₂R¹¹, C(=O)NR¹²R¹³, C(=O)NR²¹OR¹⁴, NR²¹S(O)₂R¹¹, S(O)₂NR^{12A}R^{13A}, and PO(OR²¹)₂;
R² is furyl, thienyl, a 5-membered heteroaryl group containing 1-2 nitrogen atoms, or triazolyl;
wherein said R² groups are optionally substituted with an R²⁰ group;
R¹¹ at each occurrence is independently C₁-C₆ alkyl;
R¹² and R¹³ at each occurrence are each independently selected from H and C₁-C₆ alkyl, or R¹² and R¹³, together with the nitrogen to which they are attached, form a 5-6 membered heterocycloalkyl;
wherein said alkyl and heterocycloalkyl groups are optionally substituted with an R²⁰ group;
R^{12A} and R^{13A} at each occurrence are each independently selected from H and C₁-C₆ alkyl;
R¹⁴ at each occurrence is independently C₁-C₆ alkyl;
R¹⁵ at each occurrence is independently selected from C₁-C₆ alkyl, and 5-membered heteroaryl;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and phenyl;
R²³ and R²⁴ at each occurrence are each independently selected from H and C₁-C₆ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
p is 1, 2, 3, or 4;
q is 1 or 2;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
2) when X is not present, then R¹ cannot be pyrrolidinyl, piperidinyl, or morpholinyl;
and the stereoisomeric forms, mixtures of stereoisomeric forms or pharmaceutically acceptable salts forms thereof.

3. The compound of claim 2 wherein
Y is selected from:
a) C₁-C₄ alkylene-R¹;
b) C₁-C₄ alkylene-R²;
c) C₁-C₄ alkylene-O(CH₂)ₚOR²¹,
d) CH₂C(OH)(CH₃)₂, CH₂C(CH₃)₂OH, CH₂C(OH)₂CF₃, CH₂C(OH)(C≡CH)₂, or CH₂CH(OH)CH₃,
e) CH₂CH=CH₂, or CH₂C(=C)CH₃ except when X is a bond and q is 2;
wherein said alkylene groups are optionally substituted with an R²⁰ group;
R¹ is selected from pyrrolidinyl, piperidinyl, morpholinyl, NR²¹C(=O)R¹⁴, C(=O)R¹⁵, CO₂R¹¹, C(=O)NR¹²R¹³, C(=O)NR²¹OR¹⁴, NR²¹S(O)₂R¹¹, S(O)₂NR^{12A}R^{13A}, and PO(OR²¹)₂;
R² is furyl, thienyl, or triazolonyl;
wherein said R² groups are optionally substituted with an R²⁰ group;
R¹¹ at each occurrence is independently C₁-C₄ alkyl;
R¹² and R¹³ at each occurrence are each independently selected from H and C₁-C₄ alkyl, optionally substituted with C(=O)NR^{12A}R^{13A}, or R¹² and R¹³, together with the nitrogen to which they are attached, form a pyrrolidinyl or piperidinyl ring;
R^{12A} and R^{13A} at each occurrence are each independently selected from H and C₁-C₄ alkyl;
R¹⁴ at each occurrence is independently C₁-C₄ alkyl;
R¹⁵ at each occurrence is independently selected from C₁-C₄ alkyl, and thienyl;
R²⁰ at each occurrence is independently selected from F, Cl, Br, I, OR²¹, OR²⁵, NR²³R²⁴, NHOH, NO₂, CN, CF₃, =O, C(=O)R²², CO₂R²¹, C(=O)NR²³R²⁴ or NR²¹C(=O)R²²;
R²¹ at each occurrence is independently selected from H and C₁-C₄ alkyl;
R²² at each occurrence is independently C₁-C₄ alkyl;
R²³ and R²⁴ at each occurrence are each independently selected from H and C₁-C₄ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
p is 1, 2, 3, or 4;
q is 1 or 2.

4. The compound of claim 1 having a structure of formula (V): 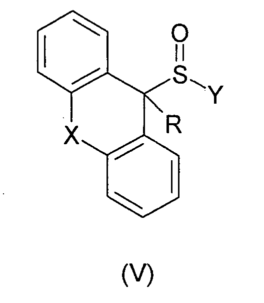 wherein
the phenylene rings are each independently optionally substituted with one to three R²⁰ groups;
X is a bond, O, S(O)_{y}, NR¹⁰, C₂ alkylene, or C₂ alkenylene, wherein said alkylene and alkenylene groups are optionally substituted with an R²⁰ group;
R is H or C₁-C₄ alkyl;
Y is selected from:
a) C₁-C₆ alkylene-R¹;
b) CH₂CR²¹=C(R²¹)₂ CH₂CR²¹=C(R²¹)₂ except when X is a bond and q is 2;
R¹ is selected from pyrrolidinyl, piperidinyl, morpholinyl, NR²¹C(=O)R¹⁴, C(=O)NR¹²R¹³, and NR²¹S(O)₂R¹¹;
R¹⁰ is independently selected from H, C₁-C₆ alkyl, C₆-C₁₀ aryl, C(=O)R¹⁴, and S(O)_{y}R¹⁴;
wherein said alkyl and aryl groups are optionally substituted with one to three R²⁰ groups;
R¹¹ at each occurrence is independently C₁-C₆ alkyl;
R¹² and R¹³ at each occurrence are each independently selected from H and C₁-C₆ alkyl, or R¹² and R¹³, together with the nitrogen to which they are attached, form a 5-6 membered heterocycloalkyl;
wherein said alkyl and heterocycloalkyl groups are optionally substituted with an R²⁰ group;
R¹⁴ at each occurrence is independently C₁-C₆ alkyl;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and phenyl;
R²³ and R²⁴ at each occurrence are each independently selected from H and C₁-C₆ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
y is 0, 1, or 2;
with the following proviso:
1) when R = H and Y is (C₁-C₆ alkylene)-C(=O)NR¹²R¹³, then the alkylene group must be substituted with a spirocycloalkyl group;
and the stereoisomeric forms, mixtures of stereoisomeric forms or pharmaceutically acceptable salts forms thereof.

5. The compound of claim 4 wherein the phenylene rings are each independently optionally substituted with one to three R²⁰ groups;
q is 1;
X is a bond;
Y is selected from:
a) C₁-C₄ alkylene-R¹;
b) CH₂CH=CH₂, or CH₂C(=C)CH₃ except when X is a bond and q is 2;
R¹ is selected from pyrrolidinyl, piperidinyl, morpholinyl, NR²¹C(=O)R¹⁴, C(=O)NR¹²R¹³, and NR²¹S(O)₂R¹¹;
R¹¹ at each occurrence is independently C₁-C₄ alkyl;
R¹² and R¹³ at each occurrence are each independently selected from H and C₁-C₄ alkyl, optionally substituted with C(=O)NR^{12A}R^{13A}, or R¹² and R¹³, together with the nitrogen to which they are attached, form a pyrrolidinyl or piperidinyl ring;
R^{12A} and R^{13A} at each occurrence are each independently selected from H and C ₁-C₄ alkyl;
R¹⁴ at each occurrence is independently C₁-C₄ alkyl;
R²⁰ at each occurrence is independently selected from F, Cl, Br, I, OR²¹, OR²⁵, NR²³R²⁴, NHOH, NO₂, CN, CF₃, =O, C(=O)R²², CO₂R²¹, C(=O)NR²³R²⁴, or NR²¹C(=O)R²²;
R²¹ at each occurrence is independently selected from H and C₁-C₄ alkyl;
R²² at each occurrence is independently C₁-C₄ alkyl;
R²³ and R²⁴ at each occurrence are each independently selected from H and C₁-C₄ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed.

6. The compound of claim 1 having a structure of formula (VI): 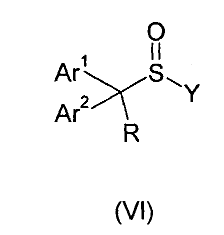 wherein
Ar¹ and Ar² are each independently phenyl optionally substituted with one to three R²⁰ groups;
R is H or C₁-C₄ alkyl;
Y is selected from:
a) C₁-C₆ alkylene-R¹;
b) C₁-C₆ alkylene-R²;
c) C₁-C₆ alkylene-O(CH₂)ₚOR²¹,
d) CH₂C(OH)(CH₃)₂, CH₂C(CH₃)₂OH, CH₂C(OH)₂CF₃, CH₂C(OH)(C≡CH)₂, or CH₂CH(OH)CH₃;
R¹ is selected from C(=O)R¹⁵, CO₂R¹¹, C(=O)NR¹²R¹³, C(=O)NR²¹OR¹⁴, S(O)₂NR^{12A}R^{13A}, and PO(OR²¹)₂;
R² is furyl, thienyl, or triazolyl; wherein said R² groups are optionally substituted with an R²⁰ group;
R¹¹ at each occurrence is independently C₁-C₆ alkyl;
R¹² and R¹³ at each occurrence are each independently selected from H and C₁-C₆ alkyl, or R¹² and R¹³, together with the nitrogen to which they are attached, form a 5-6 membered heterocycloalkyl;
R^{12A} and R^{13A} at each occurrence are each independently selected from H and C ₁-C₆ alkyl;
R¹⁴ at each occurrence is independently C₁-C₆ alkyl;
R¹⁵ at each occurrence is independently selected from C₁-C₆ alkyl, and 5-membered heteroaryl;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and phenyl;
R²³ and R²⁴ at each occurrence are each independently selected from H and C₁-C₆ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
p is 1, 2, 3, or 4;
y is 0, 1, or 2;
with the following provisos:
1) when R = H, Y is (C₁-C₆ alkylene)-R¹, and R¹ is CO₂R¹¹ or C(=O)NR¹²R¹³, then the alkylene group must be substituted with a spirocycloalkyl group;
and the stereoisomeric forms, mixtures of stereoisomeric forms or pharmaceutically acceptable salts forms thereof.

7. The compound of claim 6 wherein Ar¹ and Ar² are each independently phenyl optionally substituted with one to three R²⁰ groups;
R is H or C₁-C₄ alkyl;
Y is selected from:
a) C₁-C₄ alkylene-R¹;
b) C₁-C₄ alkylene-R²;
c) CH₂CH₂O(CH₂)₂OCH₃,
d) CH₂C(OH)(CH₃)₂, CH₂C(CH₃)₂OH, CH₂C(OH)₂CF₃, CH₂C(OH)(C≡CH)₂, or CH₂CH(OH)CH₃,
R¹ is selected from C(=O)R¹⁵, CO₂R¹¹, C(=O)NR¹²R¹³, C(=O)NR²¹OR¹⁴, S(O)₂NR^{12A}R^{13A}, and PO(OR²¹)₂;
R² is furyl, thienyl, or triazolonyl;
R¹¹ at each occurrence is independently C₁-C₄ alkyl;
R¹² and R¹³ at each occurrence are each independently selected from H and C₁-C₄ alkyl;
R^{12A} and R^{13A} at each occurrence are each independently selected from H and C₁-C₄ alkyl;
R¹⁴ at each occurrence is independently C₁-C₄ alkyl;
R¹⁵ at each occurrence is independently selected from C₁-C₄ alkyl, and thienyl;
R²⁰ at each occurrence is independently selected from F, Cl, Br, I, OR²¹, OR²⁵, NR²³R²⁴, NHOH, NO₂, CN, CF₃, C₃-C₆ spirocycloalkyl, =O, C(=O)R²², CO₂R²¹, C(=O)NR²³R²⁴, or NR²¹C(=O)R²²;
R²¹ at each occurrence is independently selected from H and C₁-C₄ alkyl;
R²² at each occurrence is independently C₁-C₄ alkyl;
R²³ and R²⁴ at each occurrence are each independently selected from H and C₁-C₄ alkyl, or R²³ and R²⁴, together with the nitrogen to which they are attached, form a 5-6 membered heterocycloalkyl;
R²⁵ at each occurrence is independently the residue of an amino acid after the hydroxyl group of the carboxyl group is removed;
with the following proviso:
1) when R = H, Y is (C₁-C₄ alkylene)-R¹, and R¹ is CO₂R¹¹ or C(=O)NR¹²R¹³, then the alkylene group must be substituted with a spirocycloalkyl group.

8. The compound of claim 1 selected in accordance with Tables 1 and 2.

9. A method of treating excessive sleepiness associated with narcolepsy, obstructive sleep apnea or shift work disorder; Parkinson's disease; Alzheimer's disease; attention deficit disorder; attention deficit hyperactivity disorder; depression; or fatigue in a subject in need thereof comprising administering to said subject a therapeutically effective amount of a compound of formula (A): 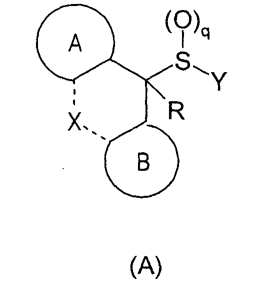 wherein
rings A and B, together with the carbon atoms to which they are attached, are each independently selected from:
a) a 6-membered aromatic carbocyclic ring in which from 1 to 3 carbon atoms may be replaced by hetero atoms selected from oxygen, nitrogen and sulfur; and
b) a 5-membered aromatic carbocyclic ring in which either:
i) one carbon atom may be replaced with an oxygen, nitrogen, or sulfur atom;
ii) two carbon atoms may be replaced with a sulfur and a nitrogen atom, an oxygen and a nitrogen atom, or two nitrogen atoms; or
iii) three carbon atoms may be replaced with three nitrogen atoms, one oxygen and two nitrogen atoms, or one sulfur and two nitrogen atoms;
wherein said rings are optionally substituted with one to three R²⁰ groups;
X is not present, is a bond, O, S(O)_{y}, NR¹⁰, C₂ alkylene, C₂₋₃ alkenylene, C(=O), C(R²¹)₂NR¹⁰, C(R²¹)=N, N=C(R²¹), C(=O)N(R¹⁰), or NR¹⁰C(=O); wherein said alkylene and alkenylene groups are optionally substituted with one to three R²⁰ groups;
R is H, C₁-C₆ alkyl, C₆-C₁₀ aryl, 5-6 membered heteroaryl, C₃-C₇ cycloalkyl, or 3-7 membered heterocycloalkyl; with the proviso that R cannot be H when R¹ is C(=O)NR¹²R¹³;
Y is C ₁-C₉ alkylene-R¹, wherein one or two carbon atoms can be replaced by one or two O, NR¹⁰, or S(O)_{y} groups, or a carbon atom can be replaced by a C₆-C₁₀ arylene, 5-10 membered heteroarylene, C₃-C₆ cycloalkylene, or 3-6 membered heterocycloalkylene group; C₂-C₆ alkenylene-R¹; or C₂-C₆ alkynylene-R¹; wherein said alkylene, alkenylene, alkynylene, arylene, heteroarylene, cycloalkylene, and heterocycloalkylene groups are optionally substituted with one to three R²⁰ groups;
R¹ is selected from H, NR¹²R¹³, NR²¹C(=O)R¹⁴, C(=O)R¹⁵, CO₂R¹¹, OC(=O)R¹¹, C(=O)NR¹²R¹³, C(=O)NR²¹OR¹⁴, C(=NR¹¹)NR¹²R¹³, NR²¹S(O)₂R¹¹, S(O)₂NR¹²R¹³, NR²¹S(O)₂NR¹²R¹³, and PO(OR²¹)₂;
R¹⁰ and R^{10A} at each occurrence are independently selected from H, C₁-C₆ alkyl, C₆-C₁₀ aryl, C(=O)R¹⁵, and S(O)_{y}R¹⁴; wherein said alkyl and aryl groups are optionally substituted with one to three R²⁰ groups;
R¹¹ at each occurrence is independently selected from H, and C₁-C₆ alkyl; wherein said alkyl is optionally substituted with one to three R²⁰ groups;
R¹² and R¹³ at each occurrence are each independently selected from H, C₁-C₆ alkyl, and C₆-C₁₀ aryl, or R¹² and R¹³, together with the nitrogen to which they are attached, form a 3-7 membered heterocycloalkyl ring;
wherein said alkyl and aryl groups and heterocycloalkyl ring are optionally substituted with one to three R²⁰ groups;
R¹⁴ at each occurrence is independently selected from C₁-C₆ alkyl, C₆-C₁₀ aryl, and arylalkyl; wherein said alkyl, aryl and arylalkyl groups are optionally substituted with one to three R²⁰ groups;
R¹⁵ at each occurrence is independently selected from C₁-C₆ alkyl, C₆-C₁₀ aryl, arylalkyl, and heteroaryl; wherein said alkyl, aryl, arylalkyl, and heteroaryl groups are optionally substituted with one to three R²⁰ groups;
R²⁰ at each occurrence is independently selected from F, Cl, Br, I, OR²¹, OR²⁵, NR²³R²⁴, NHOH, NO₂, CN, CF₃, C₁-C₆ alkyl, C₃-C₆ spirocycloalkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²¹, OC(=O)R²², C(=O)NR²³R²⁴, NR²¹C(=O)R²², NR²¹CO₂R²², OC(=O)NR²³R²⁴, NR²¹C(=O)R²², NR²¹C(=S)R²², and S(O)_{y}R²²;
R²¹ at each occurrence is independently selected from H and C₁-C₆ alkyl;
R²² at each occurrence is independently selected from C₁-C₆ alkyl, and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocycloalkyl ring;
R²⁵ at each occurrence is independently the residue of an amino acid after the hydroxyl group of the carboxyl group is removed;
m is 0 or 1;
n is 0 or 1;
q is 0, 1, or 2;
y is 0, 1, or 2;
with the proviso that when R = H and Y is (C₁-C₆ alkylene)-C(=O)NR¹²R¹³, then the alkylene group must be substituted with a spirocycloalkyl group;
and the stereoisomeric forms, mixtures of stereoisomeric forms or pharmaceutically acceptable salts forms thereof.

10. A pharmaceutical composition comprising a compound of claim 1 in admixture with one or more pharmaceutically acceptable excipients.
